# EUROPEAN PATENT APPLICATION

(11) **EP 1 048 640 A1**
(43) Date of publication of application: **02.11.2000**
(21) Application number: 98959217.5
(22) Date of filing: 15.12.1998
(51) Int. Cl.: C07C 49/417, C07C 49/517, C07C 323/12, C07D 319/06, C07D 319/08, C07D 495/10, C07D 309/32, C07D 335/02, C07D 407/06, C07D 405/06, A61K 31/335, A61K 31/38, A61K 31/37, A61K 31/12

(54) **APOPTOSIS INHIBITORS**

(30) Priority: 15.12.1997 JP 34512597
(71) Applicant: MOCHIDA PHARMACEUTICAL CO., LTD., Shinjuku-ku Tokyo 160-8515 (JP)
(72) Inventor: KATO, Kazuo Mochida Pharmaceutical Co., Ltd., Tokyo 160-8515 (JP); SATOH, Tsutomu Mochida Pharmaceutical Co., Ltd., Tokyo 160-8515 (JP); SAITOH, Fumihiko Mochida Pharmaceutical Co., Ltd., Tokyo 160-8515 (JP); TAKIGUCHI, Kazuhiko Mochida Pharmaceutical Co. Ltd, Tokyo 160-8515 (JP); TSUDA, Toshihiko Mochida Pharmaceutical Co., Ltd., Tokyo 160-8515 (JP)
(74) Representative: HOFFMANN - EITLE
(86) International application number: JP9805663
(87) International publication number: WO9931037

(57) **Abstract**

Apoptosis inhibitors containing as the active ingredient compounds represented by general formula (I) or salts thereof, wherein W₁ represents -O-, -CH₂-, etc.; W₂ represents -CH₂-, etc.; W₃ represents -O-, -CH₂-, etc.; X represent CH, C⁻, etc.; and A represents formula (α) wherein W₄ represents -O-, -CH₂-, etc.; W₅ represents -CH₂ -, etc.; and W₆ represents -O-, CH₂ -, etc. These apoptosis inhibitors inhibit apoptosis of WC8 cells induced by Fas ligand. In animal models, they potently inhibit the progress of fulminant hepatitis and relieve alopecia caused by anticancer agents. Because of being highly safe, these apoptosis inhibitors are usable as beneficial drugs.

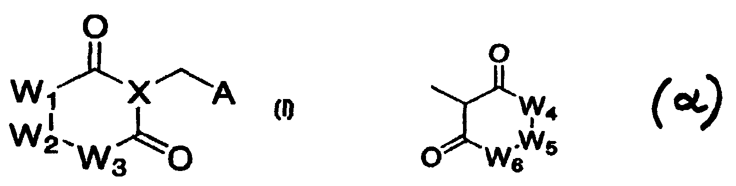

## Description

### TECHNICAL FIELD

The present invention relates to apoptosis inhibitors, compounds, pharmaceutical compositions, preventive or therapeutic agents for AIDS, preventive or therapeutic agents for fulminant hepatitis and preventive or therapeutic agents for alopecia caused by use of anticancer agents.

### BACKGROUND ART

There have been known two types of cell death different in morphology. One of them is typical death of cells, called necrosis. Another type is called apoptosis and is featured by formation of apoptotic body, fragmentation of DNA, etc. (Kerr et al., Br. J. Cancer, 265, 239 (1972)). The concept of apoptosis was originally grasped substantially as a synonym of programmed cell death. However, to date, it has been elucidated that in addition to death of cells in the process of development or normal cell turnover that have been considered as programmed death of cells, (1) anticancer agents and radiation, (2) heat shock, (3) toxins and virus infection, (4) tumor necrosis factor (TNF), (5) cellular immunity (cytotoxic T cell, natural killer cells), (6) removal of growth factor (NGF, etc.) or nutrient factor, and (7) removal of hormones or interleukins causes apoptosis.

Fas is classified into TNF receptor family, is a cell surface antigen which transmits a signal of apoptosis to cells. Human Fas is a I-type membrane protein with a molecular weight of 45 KDa having a sugar chain which consists of 319 amino acids. Fas ligand is classified into TNF family and is molecules that induces apoptosis in cells expressing Fas. Human Fas ligand is a II-type membrane protein with a molecular weight of 40 KDa which consists of 278 amino acids (Science, 267, 1449 (1995); Nippon Rinsho, 54, 1736 (1996)).

Generally, external or internal factors trigger activation of a gene that programs apoptosis. It is considered that programmed death protein is biosynthesized based on this gene and the resultant programmed death protein causes decomposition of cell itself, leading to death. However, existence of apoptosis in which new protein synthesis is not accompanied is also confirmed. Thus, presently, its detailed mechanism has not sufficiently elucidated yet.

As factors which inhibit apoptosis are known (1) physiological inhibitors (growth factor, estrogen, etc.), (2) virus gene (adenovirus E1B, Baculovirus p35, etc.), (3) pharmacological compounds (carpaine inhibitors, carcinogen, etc.) (Science, 267, 1456 (1995)). Japanese Patent Application Laid-open No. Hei 9-30983 describes that components of Chinese medicines, such as berberine. WO96/34604 describes that a Cl ion channel blocker has apoptosis inhibiting activity. Also, WO95/3054 contains a description concerning an apoptosis inhibitor having a dioxopiperazine skeleton. However, to date, no drug has been so far put on the market as an apoptosis inhibitor.

The conventional technologies relating to the compounds of the present invention include, for example, the followings.

Chem. Ber., 94, 929-47 (1961) contains a description on the synthesis of bis(2,2-dimethyl- 1,3-dioxane-4,6-dion-5-yl)methane. J. Am. Chem. Soc., 80, 5851-6 (1958) contains a description on the synthesis of bis(5,5-dimethylcyclohexane- 1,3-dion-2-yl)methane. J. Appl. Phycol., 8, 211-5 (1996) describes that addition of a solution of dimedone (5,5-dimethyl-1,3-cyclohexandione), an scavenger of formaldehyde, to an extract solution of Ascophyllum nodosum, a kind of alga resulted in formation of formaldemethone (bis(5,5-dimethylcyclohexane- 1,3-dion-2-yl) methane), which was isolated by TLC. Pathol. Oncol. Res., 1, 38-42 (1995) describes that use of dimedone as an scavenger of formaldehyde produced accompanying the apoptosis of human thyroid cancer and determination of the resultant formaldemethone by OPLC. However, there is no description at all therein that suggests the compounds of the present invention have an apoptosis inhibiting activity.

Chem. Mater., 6, 822-6 (1994) contains a description on the synthesis, X-ray diffraction and NMR of 1-[(6-hydroxy-2,2 -dimethyl-4-oxo-4H-1,3-dioxin-5-yl)methyl]piperidinium, 1-[(6-hydroxy-2,2 -dimethyl-4-oxo-4H-1,3-dioxin-5-yl) methyl]pyridinium and 1-[(6-hydroxy-2,2 -dimethyl-4-oxo-4H-1,3-dioxin-5-yl)methyl]morpholinium, which are stable precursors of methylene Meldrum's acid. U.S. Patent No: 5,243,053 contains a description on 1-[(6-hydroxy-2,2-dimethyl-4-oxo-4H-1,3-dioxin-5-yl)methyl]piperidinium as a stable precursor of methylene Meldrum's acid useful as a dienophile.

In any of the above technical contents, there is no disclosure that the compounds of the present invention have physiological activity.

### OBJECT OF THE INVENTION

It is suggested that apoptosis is related to various physiological phenomena and diseases. It has been desired a drug which, by inhibition of apoptosis, enables prophylaxis of various diseases, relieving of symptoms thereof, prevention of aggravation or therapy thereof caused by acceleration of apoptosis or abnormal termination of apoptosis or overexpression of apoptosis. The present invention provides such a drug.

The apoptosis inhibitors of the present invention can be used against various diseases described below. Concrete explanation will be made on some of problems that the present invention is to solve.

It has been known that with the use of chemotherapeutics for cancers, alopecia occurs as a side effect and thus, various attempts have been made for preventing or treating alopecia accompanying chemotherapy of cancers. Since alopecia accompanying chemotherapy of cancers is not a side effect that directly affects the life of a patient, co-medicals tend to treat it slightly. However, it is a serious problem to the patients and raises a big problem in the field of medicine, for example, some patients refuse the therapy with anticancer agents because of alopecia. Thus far, a head-cooling method and various drugs have been tried to prevent alopecia accompanied by chemotherapy of cancer but no sufficient result has been obtained. More particularly, the head-cooling method has been reported to effectively prevent alopecia when a single anticancer agent is used at low doses. In contrast, when a high dose of anticancer agent has been used or a multiple agents have been used in combination, no effective alopecia preventing effect can be obtained. Further, a possibility is suggested that this method could promote the metastasis of a cancer to the scalp and no clear denial has been made on this point. Attempts to prevent alopecia accompanied by chemotherapy of cancers with a drug include use of activated type vitamin D3, Imuvert, etc. and clinical tests have been performed, but no alopecia preventing effect has been observed. In any rate, clinically, no means for preventing or treating alopecia accompanying use of anticancer agents has been established yet.

AIDS (acquired immunodeficiency syndrome) is infection with HIV, a kind of human retrovirus and causes cellular immunity deficiency, and the patient is seriously recuperating unsatisfactorily. Since its report in 1981 in the United States of America, the number of patients increases rapidly, causing a big social problem. As a remedy in our country (Japan), azidothymidine and other nucleoside-based reverse transcriptase inhibitors and other HIV protease inhibitors such as indinavir have been known. The former have been known to cause serious side effects such as digestive organ syndrome (vomiting, etc.), myelopathy, etc. and the latter raises a problem that the frequency of developing side effect is very high. Although some improvement is observed by highly active anti-retroviral therapy (HAART) as compared with use of a single agent, there are many cases where no response to drugs is obtained because of advanced stage, ineffective cases where drug resistance is acquired, cases where continuous administration is difficult because of side effects and therefore, at present not always satisfactory results are obtained.

Fulminant hepatitis occupies several percentages of acute hepatitis and is very unfavorable disease the patient of which is seriously recuperating unsatisfactorily because as its name indicates, the patient falls in a multiorgan insufficiency and dies in a short period such as from several days to several weeks, and no effective therapy has been established yet. As the mechanism of its onset, there has been a report that it is due to mutant virus having hepatotoxicity or participation of host's excessive immune reaction has been suggested. Recently, there has been a report that administration of anti-Fas antibody onsets fulminant hepatitis-like sympton based on apoptosis and with this being a clue, the relationship between fulminant hepatitis and apoptosis has been suggested.

Under the circumstances, it has been desired to develop drugs that prevent or treat various diseases to which conventional therapy and remedy are ineffective, without causing side effects.

Not limited to the above specific examples, generally, when compounds are developed as medicines, there are many problems to be overcome from various points of view as well as potency of pharmacological activity. More particularly, safety, solubility of a base, stability and absorbability of preparation, interaction with other agents, bioavailability, etc. are considered. In particular, as for safety, not only the compound must be low in lethal dose, but also various parameters such as whether or not teratogenicity, carcinogenicity, induction of enzymes or pain to patients is present must be carefully studied, and these are problems that all the drugs have to overcome.

That is, development of drugs has been desired that not only have potent apoptosis inhibiting activity but also can be easily used by patients without feeling a pain and anxiety, are high in safety, causing no harm by interaction with other agents, and are excellent in pharmaceutical properties such as delivery to the affected part, retention, absorbability, and stability.

The present invention has for its object to solve at least one of the above problems.

### DISCLOSURE OF THE INVENTION

The present inventors have made intensive study with view to obtaining compounds having apoptosis inhibiting activity using inhibitory activity of Fas ligand induction apoptosis as indication and as a result, the inventors have found that the compound of the formula (I) have apoptosis inhibiting activity, thus accomplishing the present invention.

First aspect of the present invention is an apoptosis inhibitor characterized by containing a compound of the formula (I): 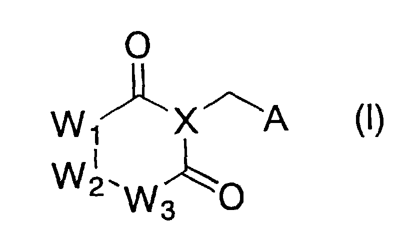 (wherein W₁ represents -O- or -CH(R₁)-, W₂ represents -S- or -CR₂(R₃)-, W₃ represents -O- or -CR₄R₅)-, R₁, R₄ and R₅ each independently represent -H or a straight chain or branched alkyl group having 1 to 6 carbon atoms, R₂ and R₃ each independently represent -H, a straight chain or branched alkyl group having 1 to 6 carbon atoms, a straight chain or branched alkyl group having 1 to 6 carbon atoms substituted by a straight chain or branched alkoxy group having 1 to 6 carbon atoms, or -(CH₂)₁O(CH₂)ₘY(CH₂)ₙH, or R₂ and R₃ together represents an alkylene having 4 to 7 carbon atoms, which may have a branched chain, or -(CH₂)ₚS(CH₂)_{q}-, Y represents O or S, l, m, n, p and q each independently represent an integer of 1 to 6, X represents CH or C⁻, A represents 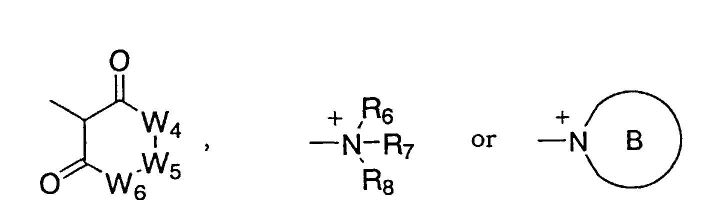 W₄ represents -O- or -CH(R₉)-, W₅ represents -S- or -CR₁₀(R₁₁)-, W₆ represents -O- or -CR₁₂(R₁₃)-, R₆, R₇ and R₈ each independently represent -H, a straight chain or branched alkyl group having 1 to 6 carbon atoms or a straight chain or branched hydroxyalkyl group having 1 to 6 carbon atoms, R₉, R₁₂ and R₁₃ each independently represent -H or a straight chain or branched alkyl group having 1 to 6 carbon atoms, R₁₀ and R₁₁ each independently represent -H, a straight chain or branched alkyl group having 1 to 6 carbon atoms, a straight chain or branched alkyl group having 1 to 6 carbon atoms substituted by a straight chain or branched alkoxy group having 1 to 6 carbon atoms, or -(CH₂)ᵣO(CH₂)ₛZ(CH₂)ₜH, or R₁₀ and R₁₁ taken together represents an alkylene having 4 to 7 carbon atoms, which may have a branched chain, or -(CH₂)ᵤS(CH₂)ᵥ-, Z represents O or S, r, s, t, u and v each independently represent an integer of 1 to 6, 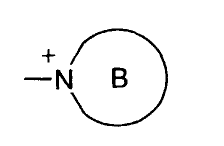 represents saturated or unsaturated 4- to 7-membered monocyclic heterocyclic onium group having 3 to 6 carbon atoms and 0 or 1 oxygen atom and 1 or 2 nitrogen atom as ring-constituting atoms which may have -CH₃, -CH₂OH or -CHO as a substituent on the ring) or salts thereof as an active ingredient. As the apoptosis inhibitor, those in which W₁ represents -O- or -CH(R₁)-, W₂ represents -S- or -CR₂(R₃)-, W₃ represents -O- or CR₄(R₅)-, R₁, R₄ and R₅ each independently represent -H or -CH₃, R₂ and R₃ each independently represent -H, -CH₃, -C₂H₅, -CH₂OCH₃, -CH₂OCH₂CH₃, -CH₂OCH₂OCH₃ or -CH₂OCH₂SCH₃ or R₂ and R₃ taken together represents -(CH₂)₄-, -(CH₂)₅-, -(CH₂)₂S(CH₂)₂- or -CH₂CH(CH₃)CH(CH₃)CH₂-, X represents CH or C⁻,
A represents 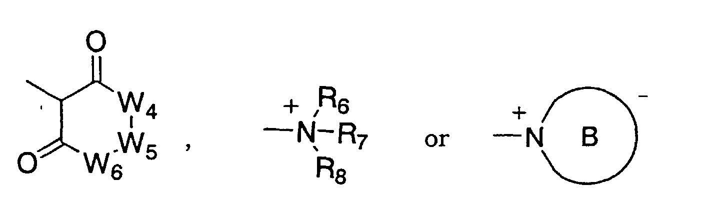 W₄ represents -O- or -CH(R₉)-, W₅ represents -S- or -CR₁₀(R₁₁)-, W₆ represents -O- or -CR₁₂(R₁₃)-, R₆, R₇ and R₈ each independently represent -H, a straight chain or branched alkyl group having 1 to 6 carbon atoms or a straight chain or branched hydroxyalkyl group having 1 to 6 carbon atoms, R₉, R₁₂ and R₁₃ each independently represent -H or -CH₃, R₁₀ and R₁₁ each independently represent -H, -CH₃, -C₂H₅, -CH₂OCH₃, -CH₂OCH₂CH₃, -CH₂OCH₂OCH₃ or -CH₂OCH₂SCH₃, or R₁₀ and R₁₁ together represents -(CH₂)₄-, -(CH₂)₅-, -(CH₂)₂S(CH₂)₂-, or -CH₂CH 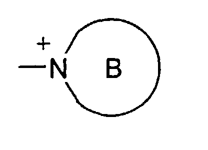 (CH₃)CH(CH₃)CH₂-, represents a saturated or unsaturated 4- to 7-membered monocyclic heterocyclic onium group having 3 to 6 carbon atoms and 0 or 1 oxygen atom and 1 or 2 nitrogen atom as ring constituting atoms which may have -CH₃, -CH₂OH or -CHO as a substituent on the ring, are preferred.

Further, those in which X is CH, A is 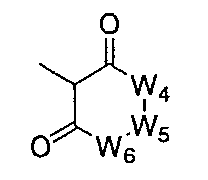 (wherein W₄, W₅ and W₆ have the same meanings as defined above) are preferred. In particular, those in which W₁, W₃, W₄ and W₆ are -O-, W₂ is -CR₂(R₃)- and W₅ is -CR₁₀(R₁₁)- are preferred. More preferred are those in which R₂, R₃, R₁₀ and R₁₁ each independently represent -CH₃ or -C₂H₅, or R₂ and R₃, or R₁₀ and R₁₁, together, represent -(CH₂)₄-, -(CH₂)₅-, -(CH₂)₂S(CH₂)₂- or -CH₂CH(CH₃)CH(CH₃)CH₂-, or those in which W₁ is -CH(R₁)-, W₂ is -S- or -CR₂(R₃)-, W₃ is -CR₄(R₅)-, W₄ is -O- or -CH(R₉)-, W₅ is -S- or -CR₁₀(R₁₁)-, and W₆ is -CR₁₂(R₁₃)-. Still more preferred are those in which R₁, R₄, R₅, R₉, R₁₂ and R₁₃ each independently represent -H or -CH₃, R₂, R₃, R₁₀ and R₁₁ each independently represent -H, -CH₃, -C₂H₅, -CH₂OCH₃, -CH₂OCH₂CH₃, -CH₂OCH₂OCH₃ or -CH₂OCH₂SCH₃.

As the apoptosis inhibitor, those in which X is C⁻, A is 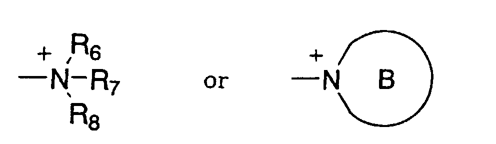 (wherein R₆, R₇, R₈ and 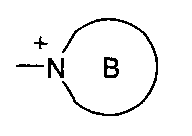 have the same meanings as defined above) are preferred. In particular, those in which W₁ and W₃ are -O-, W₂ is -CR₂(R₃)- are preferred.

More preferred are those in which A is 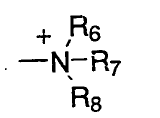 R₆ and R₇ are -CH₃, R₈ is -H, -CH₃ or a 2-hydroxyethyl group, or those in which A is

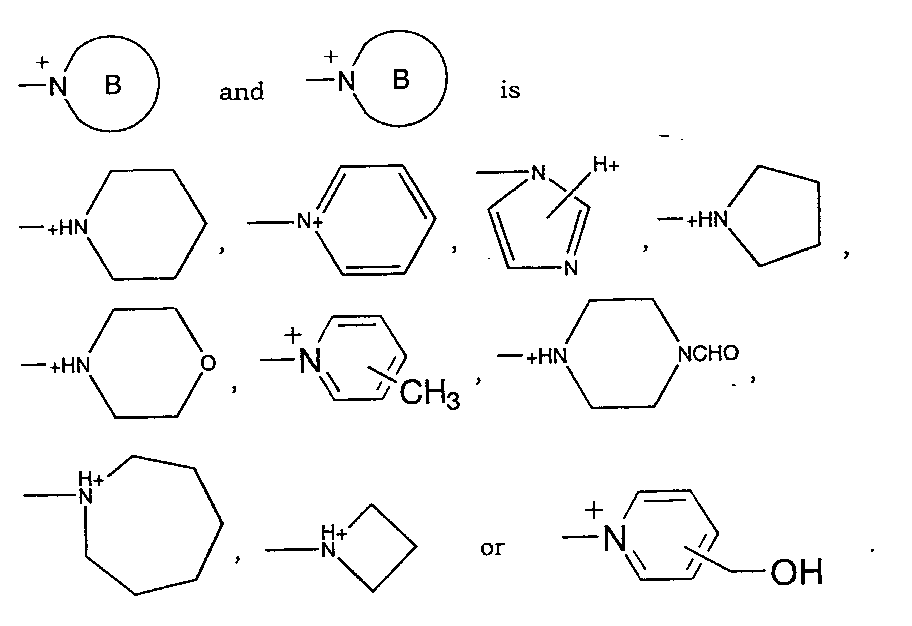

The second aspect of the present invention is a compound of the formula (Ia): 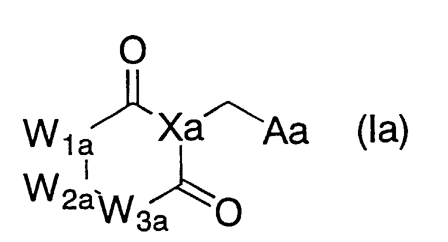 (wherein Xa represents CH or C⁻ and Aa represents 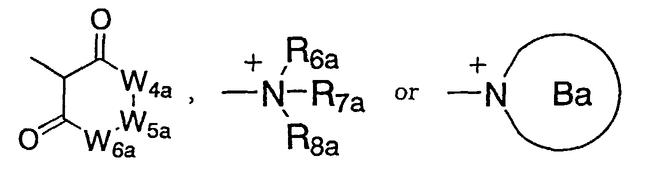 when Xa is CH and Aa is 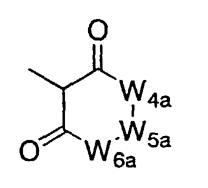 W₁ₐ, W₃ₐ, W₄ₐ and W₆ₐ represents -O-, W₂ₐ represents -CR₂ₐ(R₃ₐ)-, W₅ₐ represents CR₁₀ₐ(R₁₁ₐ)-, R₂ₐ and R₃ₐ, and R₁₀ₐ and R₁₁ₐ, together represent -(CH₂)₂S(CH₂)₂- or W₁ₐ represents -CH(R₁ₐ)-, W₂ₐ represents -S- or -CH(R₃ₐ)-, W₃ₐ represents CR₄ₐ(R₅ₐ)-, W₄ₐ represents -CH(R₉ₐ)-, W₅ₐ represents -S- or -CH(R₁₁ₐ)-, W₆ₐ represents -CR₁₂ₐ(R₁₃ₐ)-, R₁ₐ, R₄ₐ, R₅ₐ, R₉ₐ, R₁₂ₐ and R₁₃ₐ each independently represent -H or -CH₃, R₃ₐ and R₁₁ₐ each independently represent -CH₂OCH₃, -CH₂OCH₂CH₃, -CH₂OCH₂OCH₃ or -CH₂OCH₂SCH₃, and when Xa is C⁻ and Aa is 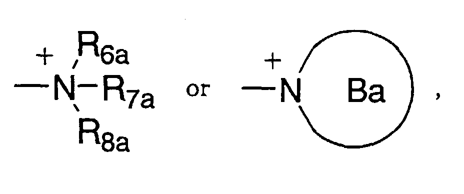 R₆ₐ, R₇ₐ and R₈ₐ each independently represent a hydrogen atom, an alkyl group having 1 to 6 carbon atoms or a hydroxyalkyl group having 1 to 6 carbon atoms, 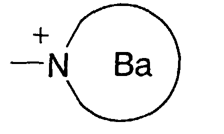 represents a saturated or unsaturated 4-, 5- or 7-membered monocyclic heterocyclic onium group having 3 to 6 carbon atoms and 0 or 1 oxygen atom and 1 or 2 nitrogen atoms as ring constituting atoms) or salts thereof.

As the compound or salts thereof, those in which Xa is CH and Aa is 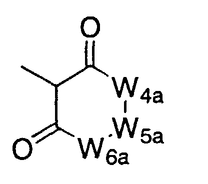 (wherein W₄ₐ, W₅ₐ, and W₆ₐ have the same meanings as defined above) or those in which Xa is C⁻ and Aa is 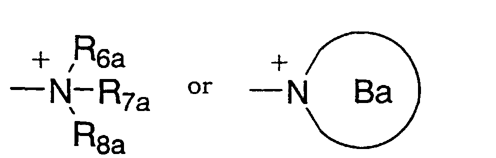 (wherein R₆ₐ, R₇ₐ, R₈ₐ and 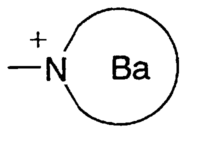 have the same meanings as defined above) are preferred.

A third aspect of the present invention relates to a pharmaceutical composition characterized by containing a compound of the formula (I): 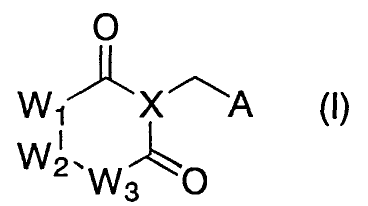 (wherein W₁, W₂, W₃, X and A have the same meanings as defined above) or salts thereof as an active ingredient.

A fourth aspect of the present invention relates to a preventive or therapeutic agent for AIDS, characterized by containing a compound of the formula (I) or salts thereof as an active ingredient.

A fifth aspect of the present invention relates to a preventive or therapeutic agent for fulminant hepatitis, characterized by containing a compound of the formula (I) or salts thereof as an active ingredient.

A sixth aspect of the present invention relates to a preventive or therapeutic agent for alopecia accompanying use of an anticancer agent (carcinostatic), characterized by containing a compound of the formula (I) or salts thereof as an active ingredient.

A seventh aspect of the present invention relates to a preventive or therapeutic agent for alopecia accompanying use of an anticancer agent, characterized by containing a Fas-mediated apoptosis inhibitor as an active ingredient.

An eighth aspect of the present invention relates to a reagent using an apoptosis inhibitor, characterized by containing a compound of the formula (I) or salts thereof as an active ingredient.

The fourth to sixth aspects of the present invention include aspects of methods for preventing or treating respective diseases by using a pharmaceutical composition characterized by containing a compound of the formula (I) or salts thereof as an active ingredient or use of a compound of the formula (I) or salts thereof for the production of a pharmaceutical composition for preventing or treating respective diseases.

The seventh aspect includes aspects of a method for preventing or treating alopecia accompanying use of an anticancer agent by using a pharmaceutical composition containing a Fas-mediated apoptosis inhibitor as an active ingredient or use of a Fas-mediated apoptosis inhibitor for the production of a pharmaceutical composition for preventing or treating alopecia accompanying use of an anticancer agent (carcinostatic).

The Fas-mediated apoptosis inhibitor as used herein is not particularly limited so far as it blocks the generation or transmission of a signal by Fas in any fashion and inhibits the biological action of Fas/Fas ligand system, particularly Fas-mediated apoptosis, more particularly Fas-mediated apoptosis by Fas ligand. It includes those having various mechanisms such as those which inhibit Fas ligand or the action or function of Fas, those which interact with Fas ligand extracellular region or Fas extracellular region, those which inhibit the interaction between Fas ligand and Fas, those which affect the interaction between Fas intracellular region and intracelluar factors interacting therewith, or those which inhibit the activity of intracellular factors (for example, ICE-like protease) participating in signal transmission of Fas-mediated apoptosis. However, it does not include known Fas, Fas derivatives (soluble Fas, variant of constitutive amino acids, etc.), Fas ligand and their neutralized antibodies. Preferably, it is a compound that has an activity of inhibiting apoptosis caused by Fas ligand of cells expressing Fas. More preferably, it is a compound that shows apoptosis inhibiting activity by the method described in Experiment 1 described below and, more preferably, a compound of the formula (I).

The apoptosis inhibitors of the present invention are useful for prevention, therapy or relieving of symptom of various diseases in human or animals in which apoptosis participate. More concretely, they can be used for preventing, treating or alleviating symptom of neurodegeneration diseases such as Alzheimer disease, Parkinson disease, amyotrophic lateral sclerosis (ALS), retinitis pigmentosa and cerebellar degeneration; virus infectious diseases such as HTLV infectious diseases, influenza virus infectious diseases, AIDS, ATL, viral wart and HIV infectious diseases; cardiovascular diseases such as myocarditis, chronic cardiac insufficiency and restenosis; metabolic or endocrine diseases such as diabetes, prostatic hyperplasia (atrophy), obesity, dyspituitarism and osteoporosis; skin diseases such as hyperkeratosis, psora, eczema, aging and itch; eye diseases such as cataract; ischemic diseases such as myocardiac infarction, cerebral infarction, apoplexy and ischemic reperfusion injury; liver diseases such as cirrhosis, fulminant hepatitis, toxic hepatitis, alcoholic hepatitis, viral hepatitis (hepatitis type C, hepatitis type A, hepatitis type B, hepatitis type F); kidney diseases such as nephritis, renal failure, cystic diseases of kidney, and glomerular nephritis; skeletal muscle diseases such as chronic fatigue syndrome and myodystrophia; digestive system diseases such as ulcerative colitis and diarrhea; respiratory diseases such as ARDS and bronchial asthma; diffuse lung diseases such as pulmonary fibrosis and interstitial pneumonia; inflammatory diseases such as ileitis, colitis and osteoarthritis; alopecia; wound; graft versus host diseases (GVHD); endotoxin-derived diseases such as septicemia, endotoxemia and endotoxin shock; blood dyscrasis such as aplastic anemia, myelodysplasia and sideroblastic anemia; congenital or acquired enzyme disorder such as purine metabolism disorder and thalassemia anemia, etc. Also, they can be used as a preservative for organs.

They can be used as preventive, therapeutic or alleviating agents for various side effects of chemotherapy or radiotherapy of cancers, for example, alopecia, skin disorder, neurological complication, pulmonary toxicity, cardiac toxicity, stomatitis, myelopathy, granulocytepenia, nausea/vomiting, diarrhea, etc.

The anticancer agents (carcinostatic) used for chemotherapy of cancers referred to herein include, for example, anticancer antibiotics (adriamycin, pirarubicin, epirubicin, daunorubicin, bleomycin, mitomycin C, mitoxantrone, actinomycin D, etc.), alkylating agents (cyclophosphamide, ifosfamide, busulphan, thio-TEPA, melphalan, dacarbazine, etc.), plant-derived anticancer agents (etoposide, vincristine, vindesine, vinblastine, taxol, taxotere, etc.), metabolic antagonists (cytarabine, fluorouracil, mercaptopurine, methotrexate, hydroxyurea, etc.), other anticancer agents (procarbazin, cisplatin, carboplatin, asparaginase, nimustine, ranimustine, interferons, etc.) etc. However, they are not limited to these.

The apoptosis inhibitors of the present invention can be used as a plant growth regulating agent.

The apoptosis inhibitors of the present invention can be used also as a reagent. The reagent referred to herein means widely those reagents used for utilizing apoptosis, for example, reagents used for judging participation of apoptosis in various biochemical reactions in organisms in general, preferably animals and plants or microbes or diseases. Also, mention will be made of reagents for screening compounds that promote apoptosis or control by apoptosis utilizing apoptosis inhibiting environment created in a simulating manner by addition of them to in vitro or in vivo systems. The reagents are those for utilizing apoptosis, preferably those for utilizing apoptosis which is Fas-mediated apoptosis, and more preferably those for utilizing apoptosis which is induced by Fas ligand. It is possible to constitute a kit for examining apoptosis using such a reagent.

As a specific example of the screening, for example, compounds that can promote or regulate apoptosis can be found by conducting screening in a system where apoptosis is inhibited with the compound according to the first aspect of the present invention in an environment where apoptosis can be induced by Fas ligand in cells (for example, WC8 cell) in which Fas ligand can induce apoptosis. Specific example of the constituent element of the kit includes, for example, the compound according to the first aspect of the present invention, Fas-expressed cells, cells expressing Fas ligand, medium, a suitable marker for judgment of results, etc.

The compounds to be found by the above screening are, from their nature, useful as new anticancer agents, remedy for leukemia, remedy for autoimmune diseases, etc.

In the definitions in the formula of the present invention, for example, when -CR₂(R₃)- is described, this means that the both substituents R₂ and R₃ are bonded to the same carbon atom.

The stereoisomers of the compounds of the invention will be explained. The compounds of the present invention may have stereoisomers depending on the substituents. These can readily be isolated or synthesized by known methods such as liquid chromatography using a commercially available chiral column, an optical resolution by crystallization, an asymmetric synthesis method, etc. Each isomer can be analyzed by optical rotation, or NMR. The present invention also includes as objects these optically active or inactive stereoisomeric forms and any arbitorary mixtures thereof.

### Production Process

The compounds of the present invention can be produced by the following production process or according to said production process.

In the formula (I), when A is 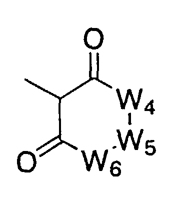 and X is CH, the compounds of the present invention can be obtained according to the following reaction scheme.

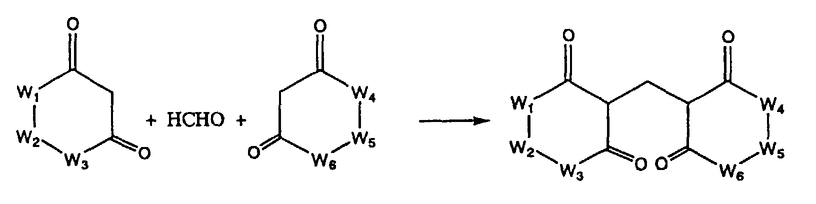

This reaction can be readily performed by heating in water or an organic solvent from room temperature to the boiling point of the solvent. The organic solvent includes alcohol solvents such as methanol and ethanol, ether solvents such as dioxane, tetrahydrofuran (THF) and diglyme, amide solvents such as dimethylformamide (DMF), dimethylacetamide and N-methylpyrrolidone. It is preferred to carry out the reaction in DMF, water or a mixed solvent thereof at a reaction temperature of from room temperature to about 100°C.

The compounds of the present invention have the following tautomers and these tautomers are also included by the present invention.

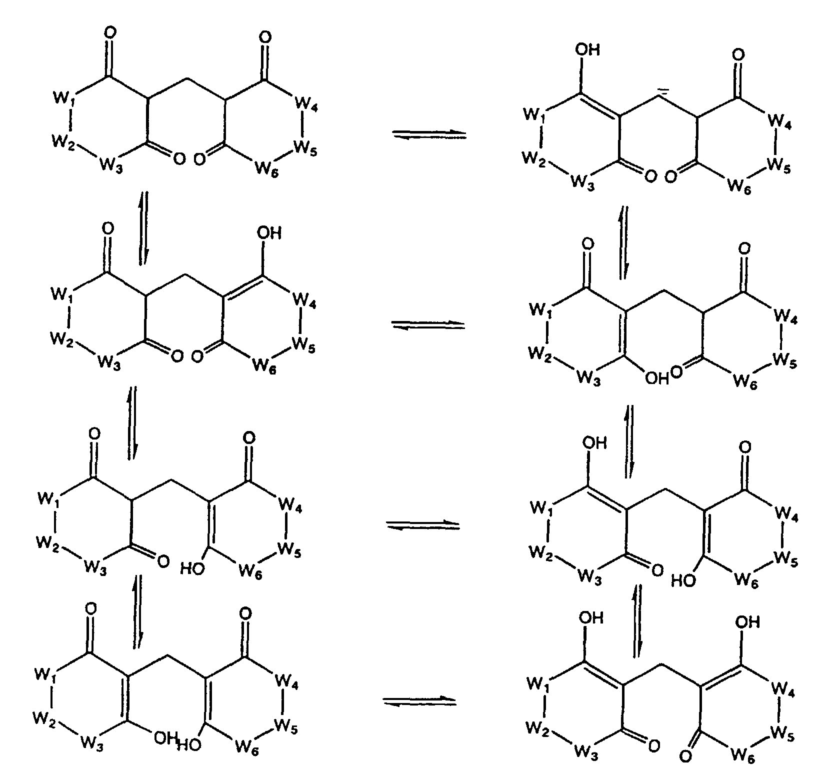

Further, when A is 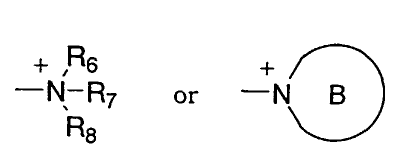 W₁ and W₃ are oxygen atoms, W₂ is -CR₂(R₃)-, R₂ and R₃ are each -CH₃ and X is C⁻, the compounds of the present invention can be obtained by reacting Meldrum's acid with an equivalent amount of formaldehyde in the presence of corresponding organic amine. The compounds of the present invention can be produced also by use of organic solvents, e.g., alcohol solvents such as methanol and ethanol, halogenated solvent such as methylene chloride and chloroform, ether solvents such as THF, dioxane, and diglyme.

The compounds of the present invention have the following tautomers and these tautomers are also included by the present invention.

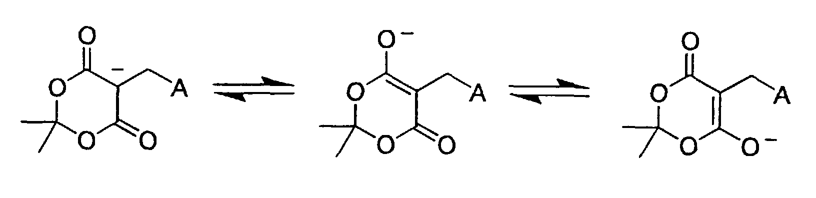

Further, the compounds in which the cation in the onium group and the anion in the dioxinedione are described in a neutralized form are also included by the present invention.

The compounds of the present invention can form salts with inorganic bases or organic bases.

Examples of these salts include alkali metal salts such as sodium salts and potassium salts, alkaline earth metal salts such as magnesium salts and calcium salts, salts with inorganic bases such as ammonium salts, salts with organic bases such as triethylamine salts, and pyridine salts, salts with basic amino acids such as arginine salts, lysine salts and histidine salts, etc. These salts are also included by the present invention.

The compounds of the present invention or salts thereof can form solvates with solvents such as water, ethanol, glycerol, etc. These solvates are also included by the present invention.

### BEST MODE FOR CARRYING OUT THE INVENTION

Next, experiment examples and examples of the present invention will be explained. However, the present invention is not limited thereto.

### Experiment Example 1 Apoptosis Inhibiting Activity

Assay of apoptosis inhibiting activity was performed according to the method of Nagata et al. (Japanese Patent Application Laid-open No. Hei 8-127594, paragraphs 0093 to 0096).

That is, the test compound was dissolved in DMSO or physiological saline to make 100 mg/mL and then adjusted to 10 µg/mL to 3000µg/mL with RPMI1640 medium (Dainippon Pharmaceutical Co., Ltd.) containing 10% fetal bovine serum. This was added in each well of 96-well flat plate (NUNC Co.) in an amount of 10 µL each. Further, transformant WC8 cell (Itoh et al., J. Immunol., 151, 621-7 (1993)) expressing human Fas antigen was suspended in RPMI1640 medium containing 10% fetal bovine serum to 5.55× 10⁵ cells/mL, and 45 µL of this suspension was added to each well. After incubation at 37°C for 30 minutes in the presence of 5% CO₂, cultivation supernatant of transformant COS-1/pM1070 (cf. WO95/ 13293, Example 21 (9)) expressing Fas ligand extracellular domain was diluted 15 folds with RPMI1640 medium containing 10% fetal bovine serum. 45 µL of this diluted solution was added to each well.

After incubation at 37°C for 18 hours in the presence of 5% CO₂, MTT assay (cf. Morten et al., J. Immunol. Methods, 119, 203-10 (1989)) was performed.

The results are shown in Table 1. In Table 1, the values indicated as IC₃₀ represents the concentration of test compounds necessary to inhibit the death of cells by apoptosis by 30%.

**Table 1**

| Compounds of Example | Apoptosis inhibiting activity IC₃₀ value (µg/mL) |
|---|---|
| Compound of Example 1 | 27.9 |
| Compound of Example 2 | 18.3 |
| Compound of Example 3 | 41.6 |
| Compound of Example 4 | 19.2 |
| Compound of Example 5 | 34.5 |
| Compound of Example 6 | 25.4 |
| Compound of Example 7 | 22.6 |
| Compound of Example 8 | 76.8 |
| Compound of Example 9 | 104.0 |
| Compound of Example 10 | 28.4 |
| Compound of Example 11 | 39.8 |
| Compound of Example 12 | 25.3 |
| Compound of Example 13 | 67.4 |
| Compound of Example 14 | 39.3 |
| Compound of Example 15 | 19.7 |
| Compound of Example 16 | 16.9 |
| Compound of Example 17 | 68.4 |
| Compound of Example 18 | 19.8 |
| Compound of Example 19 | 24.4 |
| Compound of Example 20 | 8.2 |
| Compound of Example 21 | 13.6 |
| Compound of Example 22 | 12.8 |
| Compound of Example 23 | 21.1 |
| Compound of Example 24 | 18.9 |
| Compound of Example 25 | 15.4 |
| Compound of Example 26 | 39.8 |
| Compound of Example 27 | 21.6 |
| Compound of Example 28 | 25.8 |
| Compound of Example 29 | 21.3 |
| Compound of Example 30 | 81.6 |
| Compound of Example 31 | 81.7 |
| Compound of Example 32 | 23.8 |

By addition of the compounds of the present invention, the apoptosis of WC8 cells induced by Fas ligand was inhibited.

### Experiment Example 2 Therapeutic effect on Anti-Fas Antibody-Induced Mouse Fulminant Hepatitis

The therapeutic effect on anti-Fas antibody-induced mouse fulminant hepatitis model was determined by the method of Ogasawara et al. (Nature, 364, 806-9 (1993)) with a partial modification.

That is, 5-week-old BALB/c female mice (Nippon Charles River Co., Ltd.) were orally administered with a test compound dissolved in 0.5% Tween 80 aqueous solution and after 30 minutes, anti-mouse Fas monoclonal antibody Jo2 (Pharmingen) was administered from tail vein at a dose of 3 µg/ mouse. Control was orally administered with the solvent only instead of the test compound solution. After 7 hours from the administration of the antibody, the animals were drawn blood from ocular fundus oculi (eyegrounds) and plasma was obtained by a conventional method and GPT in the plasma was measured using Fuji Dry Chem Slide GPT/ALT-P (Fuji Photo Film Co., Ltd.). The results obtained are shown in Table 2.

**Table 2 (No. 1)**

| | Dose (mg/kg) | GPT (U/L) |
|---|---|---|
| Control | - | 8180 |
| Compound of Example 1 | 30 | 5528 |
| | 100 | 5865 |
| | 300 | 3128 |

**Table 2 (No. 2)**

| | Dose (mg/kg) | GPT (U/L) |
|---|---|---|
| Control | - | 13893 |
| Compound of Example 2 | 30 | 10110 |
| | 100 | 6310 |
| | 300 | 4805 |

**Table 2 (No. 3)**

| | Dose (mg/kg) | GPT (U/L) |
|---|---|---|
| Control | - | 8173 |
| Compound of Example 20 | 30 | 6473 |
| | 100 | 5128 |
| | 300 | 2738 |

By the administration of the compounds of the present invention, an increase in value of plasma GPT in mouse fulminant hepatitis model was significantly inhibited.

### Experiment Example 3 Therapeutic effect on Alopecia in Rat with Anticancer Agent

Study of the therapeutic effect on alopecia caused by anticancer chemotherapy was performed by the method of Joaquin et al. (Cancer Res., 52, 5123-5 (1992)) with a partial modification.

That is, 5-day-old after the birth Sprague-Dawley rat (Nippon Charles River Co. Ltd.) was topically applied with the compound on its head to back everyday up to 14-day-old and 1 mg/kg of VP-16 (SIGMA) was intraperitoneally administered for 3 days continuously from 11-day-old. Control was topically applied with the solvent only instead of the test compound solution. On 21-day-old after the birth, the degree of alopecia was evaluated into 6 levels by the degree of retention of the hair on the head and back (Level 0: no alopecia was observed; Level 1: slightly thin on the head; Level 2: apparent alopecia was observed on the head; Level 3: in addition to the head, apparent alopecia was observed on the back (less than 50%); Level 4: considerable alopecia was observed on the both head and back (50% or more); Level 5: complete alopecia). The results are shown in Table 3.

**Table 3**

| | Level of alopecia | | | | | |
|---|---|---|---|---|---|---|
| Treatment | 0 | 1 | 2 | 3 | 4 | 5 |
| Control | 0 | 0 | 0 | 1 | 1 | 8 |
| Compound of Example 2 | 0 | 0 | 1 | 7 | 2 | 0 |

In the group where only the solvent was applied, complete alopecia (Level 5) was observed on the head and back in 8/10 cases after 21-day-old after the birth. In contrast, in the group in which the compound of the present invention was applied, alopecia remained Level 3 or less in 8/10 cases.

As will be apparent from the above explanation and experimental results, the compounds of the present invention have an activity of inhibiting Fas-mediated apoptosis. Further, in an animal model of fulminant hepatitis, the compounds of the present invention potently inhibited the progress of it and at the time of evaluation, no case of death at the maximum dose of a drug, thus showing high safety. In a rat model, alopecia due to anticancer agent was inhibited. That is, the experimental data inhibiting the apoptosis indicates that the compounds of the present invention are useful for preventing diseases caused by acceleration of apoptosis, or abnormal termination or overexpression of apoptosis, relieving of symptoms of the diseases, prevention of aggravation of the diseases, or therapy of the diseases.

The apoptosis inhibitors of the present invention can be provided as medicine, quasi-drugs or cosmetics.

The apoptosis inhibitors of the present invention may be obtained by appropriately combining at least one of the compounds of the formula (I) and salts thereof with a suitable pharmaceutically acceptable carrier or solvent, for example, sterilized water, physiological saline or various oils (vegetable oils, etc.), further non-toxic organic solvents or solubilizer (ethanol, acetic acid, glycerin, propylene glycol, propylene glycol dicaprylate / dicaprate, DMSO, etc.), excipients, binders, lubricants, colorants, flavourings, emulsifiers, suspending agents, gelling agents, polymers, ointment bases or cream bases (for example, hydrophilic ointment and similar cream, e.g., UNIBASE, Park Davis, etc.), lotion bases, antiseptics, perfume, stabilizers, colorants, humectants, thickeners, diluents, surfactants (for example, nonylphenoxypolyethoxyethanol (Nonoxynol-9), etc.) or components which aid the activity of the basis (for example, absorption promoter, etc.) and formulated into the following preparation forms.

Specific examples of preparation forms include, in addition to oral agents such as tablet, capsule, granule, subtilized granule, powder, suppository and syrup, injections such as aqueous or non-aqueous injections, emulsified or suspended injections, or solid injections that are dissolved, emulsified, or suspended upon use, external liquids (for example, infusion, gargle/mouse rinse solution, poultice, inhalation, air spray (aerosol), enema, fomentation, toilet, bath, disinfector, ear nose liquid, etc.), external preparations such as adhesive dressing, ointment, liniment, lotion, suppository, cream, dusting powder, liquid coating (shaking lotion, emulsified lotion), jelly, pasta and plaster, and so on. Further, liposome preparations enclosing the apoptosis inhibitors of the present invention may be made. The apoptosis inhibitors of the present inventions may be applied to shampoo, rinse, conditioner, hair tonic, hair liquid or the like.

The apoptosis inhibitors of the present invention can be administered to patients no matter whether orally or parenterally (for example, intrarectal administration, transdermal absorption, transmucosal absorption, intravenous administration, intramuscular administration, subcutaneous administration, or intradermal administration, etc.). The daily dosage in the case of oral agents, injections and suppository is 1 mg to 5000 mg, preferably 10 to 1000 mg, in terms of the above compound, and in the case of local agents (for example, external liquids, ointments, emulsion, suspensions, creams, aerosols, etc.), 0.01 to 20% preparation, preferably 0.1 to 10% preparation, is prepared and a suitable amount thereof can be dispersed, coated or sprayed. In any of them, the amount may be controlled appropriately depending on the case of patient, and on the kind and dose of a drug used in the chemotherapy when used as a preventive or therapeutic agent for side effects associated with chemotherapy. The total amount may be administered at a time or dividedly in 2 to 6 times. Alternatively, continuous administration such as instillation is also possible.

When the apoptosis inhibitors of the present invention are used as a preventive/therapeutic agent for side effects associated with chemotherapy, administration of 1 or 2 times a day is started 5 to 8 days before the chemotherapy is started. This is continued while chemotherapy is continued and is desirably continued for several days after the completion of chemotherapy. However, the administration may be substantially at the same time with the administration of the chemotherapeutic or after its administration. The apoptosis inhibitors of the present invention may be blended with other active ingredients (for example, antioxidants, antibiotics, anti-fungal agents, anti-inflammatory agents, bactericides (for example, salicylic acid, resorcinol, hexachlorophene, etc.), antiviral agents, vitamines (for example, nicotinic acid, vitamin E, vitamin A, panthothenic acid, etc.), etc.).

### EXAMPLES

Next, the present invention will be explained in detail by examples. However, the present invention is not limited to these examples. IR (infrared absorption) was measured using potassium bromide tablet or thin film and expressed in cm⁻¹. NMR (Nuclear Magnetic Resonance) was measured at 270 or 300 MHz (room temperature) using TMS (tetramethylsilane) as an internal standard and expressed in ppm.

As for the solvent used, CDCl₃ is chloroform-d or DMSO-d₆ is dimethyl sulfoxide-d₆. Multiplicity of each absorption spectrum was defined as follows. s is singlet, d is doublet, t is a triplet, q is a quadruplet, dd is a doublet of doublet, tt is a triplet of triplet, m is a multiplet, brs is broad singlet. The coupling constants are expressed in Hz.

### Example 1 Synthesis of bis(2,2-dimethyl-1,3-dioxane-4,6-dion-5-yl)methane

### Process A:

7.2 g of Meldrum's acid was added to 20 ml of N,N-dimethylformamide and dissolved at room temperature. To this solution was added 2.02 g of 37% formaldehyde aqueous solution and the mixture was left to stand at room temperature for 2 hours. 12.5 ml of water was added to the reaction mixture and colorless crystals that precipitated were collected by filtration. The resultant crystals were dissolved in 70 ml of acetone with heating and the solution was added to 140 ml of water. After left to stand overnight at room temperature, crystals that formed were collected by filtration to obtain 6.1 g of the titled compound (yield: 80%).
NMR(CDCl₃); 4.53 (2H, t, J=7.0Hz), 2.79(2H, t, J=7.0Hz), 1.85 (6H,s), 1.80 (6H,s)
Melting point; 143.0-144.0°C

### Process B:

1.44 g of Meldrum's acid was added to 5 ml of water and dissolved with heating. To this mixture was added 0.4 g of 37% formaldehyde aqueous solution and the mixture was left to stand at room temperature for 10 hours. The colorless crystals precipitated were collected by filtration and dissolved in 3 ml of acetone with heating. To the solution was added 10 ml of water and left to stand overnight at room temperature. The crystals precipitated were collected by filtration to obtain 1.0 g of the titled compound (yield 67%). The obtained compound had a melting point that coincided with that of the compound obtained by the above process A.

### Example 2 Synthesis of bis(1,3-cyclohexandion-2-yl)methane

The process A in Example 1 was followed except that 5.15 g of 1,3-cyclohexanedione was used instead of Meldrum's acid to obtain 4.0 g (yield: 68%) of the titled compound.
NMR (CDCl₃); 11.98 (2H, s), 3.14 (2H, s), 2.58-2.22 (8H, m), 2.08-1.76 (4H, m)
Melting point; 133.4-135.0°C

### Process C:

5.15 g of 1,3-cyclohexanedione was dissolved in 50 ml of pyridine at room temperature and 3.5 ml of 37% formaldehyde aqueous solution was added thereto. The mixed solution was heated at external temperature of 70°C for 2 hours and then the solvent was distilled off under reduced pressure. The residue was purified by silica gel column chromatography to obtain 1.5 g (yield: 14%) of the titled compound as colorless crystals.

The obtained compound was coincided with the compound obtained by the above process A by NMR.

### Example 3 Synthesis of bis(1,5-dioxa-9-thiaspiro[5.5]undecane-2,4-dion-3-yl)methane

### Step 1 Synthesis of 1,5-dioxa-9-thiaspiro[5.5]undecane-2,4-dione

3.12 g of malonic acid were suspended in 18 ml of acetic anhydride, to which was added 0.15 ml of concentrated sulfuric acid at 0°C and the mixture was stirred at room temperature for 30 minutes. To the reaction mixture was added 3.48 g of 4-oxothiane at 0°C and the mixture was stirred at room temperature for 5 days. After adding water at 0°C, the reaction mixture was extracted with ethyl acetate. The organic layer was washed successively with water and with brine, and then dried over sodium sulfate. Under reduced pressure, the solvent was distilled off and the residue was purified by silica gel column chromatography (hexane/ethyl acetate = 4/1 to 2/1) to obtain 2.71 g (yield: 45%) of the titled compound as yellow crystals.
NMR (CDCl₃); 3.65 (2H, s), 2.90-2.80 (4H, m), 2.35- 2.27 (4H, m)
IR (KBr); 1757, 1321, 1088, 980
Melting point; 84.6-92.8°C

### Step 2 Synthesis of bis(1,5-dioxa-9-thiaspiro[5.5]undecane-2,4-dion-3-yl)methane

300 mg of the compound obtained in the Step 1 was dissolved in 1 ml of N,N-dimethylformamide, to which was added 0.6 ml of 37% formaldehyde aqueous solution at 0 °C and the mixture was stirred at room temperature overnight. The crystals precipitated were collected by filtration to obtain 223 mg (yield: 72%) of the titled compound.
NMR(DMSO-d₆); 4.45 (2H, brs), 2.80-2.08 (18H, m)
IR (KBr); 1784, 1753, 1325, 1240, 1024
Melting point; 199.0-202.9°C

### Example 4 Synthesis of bis(2-ethyl-2-methyl- 1,3-dioxane-4,6-dion-5-yl) methane

### Step 1 Synthesis of 2-ethyl-2-methyl-1,3-dioxane-4,6-dione

The process of Example 3, Step 1 was followed except that 1.7 g of 2-butanone was used instead of 4-oxothiane to obtain 0.8 g (yield: 20%) of the titled compound as pale yellow oil.

### Step 2 Synthesis of bis(2-ethyl-2-methyl-1,3-dioxane-4,6-dion-5-yl)methane

The process of Example 1, Process B was followed except that 0.37 g of the pale yellow oil obtained in Step 1 above was used instead of Meldrum's acid to obtain 0.3 g (yield: 90%) of the titled compound as colorless crystals.

### Example 5 Synthesis of bis(trans-2,3-dimethyl-6,10-dioxaspiro[4.5]decane-7,9-dion-8-yl)methane

### Step 1 Synthesis of trans-2,3-dimethyl-6,10-dioxa-spiro[4.5]decane-7,9-dione

The process of Example 3, Step 1 was followed except that 1.12 g of trans-3,4-dimethylcyclopentanone was used instead of 4-oxothiane to obtain 1.11 g (yield: 56%) of the tided compound as pale yellow oil.

### Step 2 Synthesis of bis(trans-2,3-dimethyl-6,10-dioxaspiro[4.5]decane-7,9-dion-8-yl)methane

The process of Example 3, Step 2 was followed except that 300 mg of the pale yellow oily compound obtained in the Step 1 above instead of the compound obtained in Example 3, Step 1 to obtain 90 mg (yield: 30%) of the titled compound as pale yellow crystals.

### Example 6 Synthesis of bis(5-ethoxymethylcyclohexane-1,3-dion-2-yl)methane

### Step 1 Synthesis of 3-ethoxymethyl- 1,5-dimethoxycyclohexa- 1,4-dione

4.96 g of 60% sodium hydride were suspended to 140 ml of N,N-dimethylformamide and a solution of 21.1 g of 3-hydroxymethyl- 1,5-dimethoxycyclohexa-1,4-diene (synthesized by the method described in J. Am. Chem. Soc., 85, 41 (1963)) in 60 ml of N,N-dimethylformamide was added thereto at 0°C and the mixture was stirred at room temperature for 30 minutes. Ethyl iodide was added at 0°C and stirred for 10 minutes and further stirred at room temperature for additional 2 hours. Water was added to the reaction mixture at 0°C, and was extracted with ethyl acetate. The organic layer was successively washed with water and with brine and dried over sodium sulfate. The solvent was distilled off under reduced pressure and the resultant residue was purified by silica gel column chromatography (hexane/ethyl acetate = 20/1) to obtain 17.7 g (yield: 72%) as a pale yellow oil.
NMR (CDCl₃); 4.68 (2H, d, 3.5Hz), 3.58 (6H, s), 3.52 (2H, q, J=7.1Hz, 3.31-3.24 (2H, m), 3.28-3.16 (1H, m), 2.82-2.75 (2H, m), 1.23 (3H, t, J=7.1Hz)
IR (neat); 1695, 1443, 1398, 1207, 1149, 818

### Step 2 Synthesis of bis(5-ethoxymethylcyclohexane-1,3-dion-2-yl)methane

To a solution of 17.7 g of the pale yellow oil obtained in the Step 1 in 300 ml of tetrahydrofuran were added 200 ml of 1 N hydrochloric acid at 0°C and the mixture was stirred at room temperature for 2 hours. At 0°C, water was added to the reaction mixture, which was extracted with chloroform. The organic layer was washed with brine and dried over sodium sulfate. The solvent was distilled off under reduced pressure and the residue was dissolved in 80 ml of N,N-dimethylformamide. To this were added 3.63 ml of 37% formaldehyde aqueous solution at 0°C and the mixture was stirred at room temperature overnight. At 0°C, water was added to the reaction mixture, which was extracted with ethyl acetate. The organic layer was washed successively with water and brine and dried over sodium sulfate. Under reduced pressure, the resultant residue was purified by silica gel column chromatography (hexane/ethyl acetate = 4/1 to 2/1) to obtain 11.3 g (yield: 71%) of the titled compound as colorless crystals.
NMR (CDCl₃); 12.02 (1.3H, s), 11.74 (0.7H, s), 3.47 (4H, q, J=7.0Hz), 3.37 (4H, d, J=4.9Hz), 3.16-3.11 (2H, m), 2.59-2.15 (10H, m), 1.18 (6H, t, J=7.0Hz)
IR (KBr); 1614, 1362, 1120, 870, 604
Melting point; 72.3-77.7°C

### Example 7 Synthesis of bis(5-methoxymethylcyclohexane-1,3-dion-2-yl)methane

### Step 1 Synthesis of 1,5-dimethoxy-3-methoxymethyl-cyclohexa-1,4-diene

The process of Example 6, Step 1 was followed except that 1.0 g of methyl iodide was used instead of ethyl iodide to obtain 914 mg (yield: 91 %) of the titled compound as colorless oil.

### Step 2 Synthesis of bis(5-methoxymethylcyclohexane-1,3-dion-2-yl)methane

The process of Example 6, Step 2 was followed except that 300 mg of the colorless oil obtained in the Step 1 was used instead of the compound obtained in Example 6, Step 1 to obtain 8.4 mg (yield: 5%) of the titled compound as colorless crystals.

### Example 8 Synthesis of bis(5-methylthiomethoxymethyl-cyclohexane-1,3-dion-2-yl)methane

### Step 1 Synthesis of 1,5-dimethoxy-3-methylthiomethoxycyclohexa-1,4-diene

The process of Example 6, Step 1 was followed except that 700 mg of methylthiomethyl chloride was used instead of ethyl iodide to obtain 453 mg (yield: 35 %) of the titled compound as pale yellow oil.

### Step 2 Synthesis of bis(5-methylthiomethoxymethyl-cyclohexane- 1,3-dion-2-yl)methane

The process of Example 6, Step 2 was followed except that 200 mg of the pale yellow oil obtained in the Step 1 was used instead of the compound obtained in Example 6, Step 1 to obtain 105 mg (yield: 29%) of the titled compound as pale yellow crystals.

### Example 9 Synthesis of bis(5-methoxymethyloxymethyl-cyclohexane-1,3-dion-2-yl)methane

### Step 1 Synthesis of 1 ,5-dimethoxy-3-methyloxymethyl-cyclohexa- 1,4-diene

The process of Example 6, Step 1 was followed except that 541 mg of methoxymethyl chloride was used instead of ethyl iodide to obtain 748 mg (yield: 68 %) of the titled compound as pale yellow oil.

### Step 2 Synthesis of bis(5-methoxymethyloxymethyl-cyclohexane-1,3-dion-2-yl)methane

The process of Example 6, Step 2 was followed except that 200 mg of the pale yellow oil obtained in the Step 1 was used instead of the compound obtained in Example 6, Step 1 to obtain 70.2 mg (yield: 22%) of the titled compound as colorless crystals.

### Example 10 Synthesis of bis(4,5-dimethylcyclohexane- 1,3-dion-2-yl) methane

### Step 1 Synthesis of 4,5-dimethylcyclohexane- 1,3-dione

21.6 g of 2-butanone and 80 g of methyl crotonate were dissolved in 400 ml of anhydrous toluene at room temperature. To this mixture were added 32.6 g of sodium methoxide. After standing the mixture at room temperature overnight, 300 ml of ice water was added thereto. The water layer was removed and extracted with 100 ml of ether. The water layer was adjusted to pH about 3 with concentrated hydrochloric acid. This was extracted with 200 ml of ether three times. The ether layers were combined and washed with 200 ml of brine twice and dried over sodium sulfate. Under reduced pressure, the solvent was distilled off to obtain 42.5 g of the tided compound as pale yellow oil.
NMR (CDCl₃); 3.44 (1H, d, J=16.8Hz), 3.32 (1H, dd, J=16.8Hz, 2.0Hz), 2.90-2.76 (2H, m), 2.64-2.54 (1H, m), 2.48-2.16 (1H, m), 1.17 (3H, d, J=6.8Hz), 0.81 (3H, d, J=7.1Hz),
IR (neat); 1732, 1614, 1188, 851, 739

### Step 2 Synthesis of bis(4,5-dimethylcyclohexane-1,3-dion-2-yl)methane

The process of Example 1, Process B was followed except that 42.5 g of the oil obtained in the Step 1 above instead of Meldrum's acid to obtain 20 g (yield: 30%) of the titled compound as colorless crystals.
NMR (CDCl₃); 12.02 (1.4H, s), 11.82-11.70 (0.6H, m), 3.11 (2H, s), 2.51 (2H, dd, J=17.9Hz, 4.3Hz), 2.32-1.66 (6H, m), 1.20 (6H, d, J=6.5Hz), 1.07 (6H, d, J=6.2Hz)
IR (KBr); 2970, 1614, 1583, 1381
Melting point; 107.5-109.4°C

### Example 11 Synthesis of 2-(5,6-dihydro-4-hydroxy-6-methyl-2H-pyran-2-on-3-yl)methylcyclohexane-1,3-dione

To a solution of 175 mg of 1,3-cyclohexanedione and 200 mg of 5,6-dihydro-4-hydroxy-6-methyl-2H-pyran-2-one in 2 ml of N,N-dimethylformamide was added 0.13 ml of 37% formaldehyde aqueous solution and the mixture was stirred at room temperature for 3 hours. Water was added to the reaction mixture, which was then extracted with ethyl acetate. The organic layer was successively washed with water and with brine and dried over sodium sulfate. The solvent was distilled off under reduced pressure and the resultant residue was purified by silica gel column chromatography (hexane/ethyl acetate = 10/1) to obtain 81.7 mg (yield: 20%) of the titled compound as colorless crystals.
NMR (CDCl₃); 11.85 (1H, s), 11.73 (1H, s), 4.58-4.42 (1H, m), 3.29 (1H, d, J=15.0Hz), 3.01 (1H, d, J=15.0Hz), 2.59-2.23 (6H, m), 2.04-1.81 (2H, m), 1.44 (3H, d, J=6.0Hz)
IR (KBr); 1632, 1385, 1230, 1134, 866
Melting point; 96.0-101.7°C

### Example 12 Synthesis of (3,5-dioxothian-4-yl) methane

The process of Example 1, Process B was followed except that 0.7 g of 3,5-dioxothian (synthesized by the method described in J. Org. Chem., 42, 1163 (1977)) was used instead of Meldrum's acid to obtain 0.2 g (yield 13%) of the titled compound as pale yellow crystals.

### Example 13 Synthesis of bis(5-ethylcyclohexane-1,3-dion-2-yl)methane

The process of Example 1, Process A was followed except that 100 mg of 5-ethylcyclohexane-1,3-dione were used instead of Meldrum's acid to obtain 90.7 mg (yield: 44%) of the titled compound as colorless crystals.

### Example 14 Synthesis of bis(1,5-dioxaspiro[5.5]undecane-2,4-dion-3-yl) methane

The process of Example 1, Process B was followed except that 0.2 g of 1,5-dioxaspiro[5.5]undecane-2,4-dione was used instead of Meldrum's acid to obtain 0.3 g (yield: 22%) of the titled compound as colorless crystals.

### Example 15 Synthesis of bis(6,10-dioxaspiro[4.5]decane-7,9-dion-8-yl)methane

The process of Example 1, Process A was followed except that 100 mg of 6,10-dioxaspiro[4.5]decane-7,9-dione were used instead of Meldrum's acid to obtain 135 mg (yield: 65%) of the titled compound as colorless crystals.

### Example 16 Synthesis of bis(5-methylcyclohexane-1,3-dion-2-yl)methane

The process of Example 1, Process B was followed except that 0.5 g of 5-methylcyclohexane-1,3-dione was used instead of Meldrum's acid to obtain 0.2 g (yield: 3.8%) of the titled compound as colorless crystals.

### Example 17 Synthesis of bis(5,5-dimethylcyclohexane-1,3-dion-2-yl)methane

The process of Example 1, Process A was followed except that 1.0 g of 5,5-dimethylcyclohexane-1,3-dione was used instead of Meldrum's acid to obtain 0.91 g (yield: 88%) of the titled compound as colorless crystals.

### Example 18 Synthesis of bis(4,4-dimethylcyclohexane-1,3-dion-2-yl) methane

The process of Example 1, Process A was followed except that 500 mg of 4,4-dimethylcyclohexane-1,3-dione were used instead of Meldrum's acid to obtain 453 mg (yield: 85%) of the tided compound as colorless crystals.

### Example 19 Synthesis of bis(4,6-dimethylcyclohexane-1,3-dion-2-yl)methane

The process of Example 1, Process B was followed except that 0.14 g of 4,6-dimethylcyclohexane-1,3-dione was used instead of Meldrum's acid to obtain 0.1 g (yield: 13%) of the titled compound as colorless crystals.

### Example 20 Synthesis of 1-[(6-hydroxy-2,2-dimethyl-4-oxo-4H-1,3-dioxin-5-yl)methyl]pyridinium

To 150 ml of pyridine were added 20.0 g of Meldrum's acid and dissolved at room temperature. To the resultant pale yellow solution was added 10.4 ml of 37% formaldehyde aqueous solution at a time and the mixture was stirred for 1 hour. The solvent was distilled off under reduced pressure to obtain pale yellow crystals. These were stirred in 200 ml of hexane for 30 minutes and then collected by filtration to obtain 32.2 g (yield 98%) of the titled compound.
NMR (CDCl₃); 9.0 (2H, d, J=5.3Hz), 8.20-8.11 (1H, m), 7.78-7.69 (2H, m), 5.83 (2H, brs), 1.69 (6H, s)
Melting point; 350°C or higher.

### Example 21 Synthesis of 1-[(6-hydroxy-2,2-dimethyl-4-oxo-4H-1,3-dioxin-5-yl)methyl]-3-hydroxymethylpyridiniuim

The process of Example 20 was followed except that 1.09 g of 3-pyridinemethanol and 20 ml of ethanol were used instead of pyridine to obtain 0.9 g of the titled compound (yield 34%) as pale yellow crystals.

### Example 22 Synthesis of N-[(6-hydroxy-2,2-dimethyl-4-oxo-4H-1,3-dioxin-5-yl)methyl]-N-(2-hydroxyethyl)-N,N-dimethylammonium

The process of Example 20 was followed except that 309 mg of N,N-dimethylaminoethanol and 20 ml of ethanol were used instead of pyridine to obtain 809 mg of the titled compound (yield 95%) as colorless crystals.

### Example 23 Synthesis of 1-[(6-hydroxy-2,2-dimethyl-4-oxo-4H-1,3-dioxin-5-yl)methyl]-4-methylpyridinium

The process of Example 20 was followed except that 129 mg of picoline and 20 ml of ethanol were used instead of pyridine to obtain 242 mg of the titled compound (yield 70%) as pale yellow crystals.

### Example 24 Synthesis of N-[(6-hydroxy-2,2-dimethyl-4-oxo-4H-1,3-dioxin-5-yl)methyl]-N,N,N-trimethylammonium

The process of Example 20 was followed except that 82.2 mg of trimethylamine and 20 ml of ethanol were used instead of pyridine to obtain 255 mg of the titled compound (yield 90%) as colorless crystals.

### Example 25 Synthesis of 1-[(6-hydroxy-2,2-dimethyl-4-oxo-4H-1,3-dioxin-5-yl)methyl]imidazolinium

The process of Example 20 was followed except that 0.07 g of imidazole and 20 ml of ethanol were used instead of pyridine to obtain 0.8 g of the titled compound (yield 36%) as colorless crystals.

### Example 26 Synthesis of 1-[(6-hydroxy-2,2-dimethyl-4-oxo-4H-1,3-dioxin-5-yl)methyl]pyrrolidinium

The process of Example 20 was followed except that 98.9 mg of pyrrolidine and 20 ml of ethanol were used instead of pyridine to obtain 269 mg of the titled compound (yield 85%) as colorless crystals.

### Example 27 Synthesis of 4-formyl-1-[(6-hydroxy-2,2-dimethyl-4-oxo-4H-1,3-dioxin-5-yl)methyl]piperazinium

The process of Example 20 was followed except that 159 mg of N-formylpiperazine and 20 ml of ethanol were used instead of pyridine to obtain 290 mg of the titled compound (yield 81%) as colorless crystals.

### Example 28 Synthesis of N-[(6-hydroxy-2,2-dimethyl-4-oxo-4H-1,3-dioxin-5-yl)methyl]-N,N-dimethylammonium

The process of Example 20 was followed except that 0.7 ml of 2N methanol solution of dimethylamine and 20 ml of ethanol were used instead of pyridine to obtain 249 mg of the titled compound (yield 89%) as colorless crystals.

### Example 29 Synthesis of 1-[(6-hydroxy-2,2-dimethyl-4-oxo-4H-1,3-dioxin-5-yl)methyl]homopiperidinium

The process of Example 20 was followed except that 138 mg of homopiperidine and 20 ml of ethanol were used instead of pyridine to obtain 295 mg of the titled compound (yield 83%) as colorless crystals.

### Example 30 Synthesis of 1-[(6-hydroxy-2,2-dimethyl-4-oxo-4H-1,3-dioxin-5-yl)methyl]azetidinium

The process of Example 20 was followed except that 79.4 mg of azetidine and 20 ml of ethanol were used instead of pyridine to obtain 254 mg of the titled compound (yield 86%) as pale yellow crystals.

### Example 31 Synthesis of 1-[(6-hydroxy-2,2-dimethyl-4-oxo-4H-1,3-dioxin-5-yl)methyl]piperidinium

The process of Example 20 was followed except that 1.06 g of piperidine and 20 ml of ethanol were used instead of pyridine to obtain 2.0 g of the titled compound (yield 70%) as colorless crystals.

### Example 32 Synthesis of 1-[(6-hydroxy-2,2-dimethyl-4-oxo-4H-1,3-dioxin-5-yl)methyl]morpholinium

The process of Example 20 was followed except that 121 mg of morpholine and 20 ml of ethanol were used instead of pyridine to obtain 218 mg of the titled compound (yield 64%) as colorless crystals.

### Preparation Example

Hereinafter, preparation examples of the present invention will be described. However, the present invention is not limited to the following examples.

### Example A Tablet

| | |
|---|---|
| Compound of Example 1 | 125 g |
| Lactose | 725 g |
| Cornstarch | 120 g |
| Polyvinyl alcohol | 15 g |
| Magnesium stearate | 15 g |

After the above components were weighed, the compound of Example 1, lactose and cornstarch were mixed homogeneously and an aqueous solution of polyvinyl alcohol was added thereto. The mixture was granulated by a wet granulation method to prepare granules. The granules were dried and magnesium stearate was added thereto, followed by compression tableting to form 200-mg tablets.

### Example B Tablet

| | |
|---|---|
| Compound of Example 2 | 300 g |
| Lactose | 550 g |
| Cornstarch | 120 g |
| Polyvinyl alcohol | 15 g |
| Magnesium stearate | 15 g |

After the above components were weighed, 200-mg tablets were prepared in the same manner as in Example A.

### Example C Granule

| | |
|---|---|
| Compound of Example 6 | 200 g |
| Lactose | 150 g |
| Cornstarch | 400 g |
| Crystalline cellulose | 200 g |
| Hydroxypropyl cellulose | 50 g |

After the above components were weighed, the compound of Example 6, lactose, cornstarch and crystalline cellulose were-mixed homogeneously and an aqueous solution of hydroxypropylcellulose was added thereto. The mixture was granulated by a wet granulation method to prepare granules. The granules were dried and dressed to prepare granules.

### Example D Capsule

| | |
|---|---|
| Compound of Example 25 | 500 g |
| Lactose | 480 g |
| Magnesium stearate | 20 g |

The above components were weighed and homogeneously mixed. The mixed powder was filled in No. 1 hard capsules in an amount of 200 mg per capsule to prepare capsules.

### Example E Suppository

| | |
|---|---|
| Compound of Example 20 | 300 g |
| Polyethylene glycol 4000 | 600 g |
| Polyethylene glycol 1500 | 100 g |

The compound of Example 20 was sufficiently ground in a mortar to make fine powder and 1-g suppositories for rectum were prepared by a melting method.

### Example F Powder

| | |
|---|---|
| Compound of Example 22 | 200 g |
| Lactose | 790 g |
| Magnesium stearate | 10 g |

The above components were weighed and then homogeneously mixed to prepare 20% powder.

### Example G External liquid preparation

| | |
|---|---|
| Compound of Example 25 | 10% (w/w) |
| Nonoxinol-9 | 3% |
| Ethanol | 87% |

The above components were weighed and then mixed with nonoxinol-9 and ethanol. To this solution was added the compound of Example 25 and stirred until dissolution to prepare an external liquid preparation.

### Example H External liquid preparation

| | |
|---|---|
| Compound of Example 8 | 1% (w/w) |
| Propylene glycol | 10% |
| Propylene glycol dicaprylate/dicaprate | 30% |
| Butylated hydroxytoluene (BHT) | 0.05% |
| Butylated hydroxyanisole (BHA) | 0.05% |
| Ethanol | 58.9% |

The above components were weighed and then propylene glycol dicaprylate/dicaprate, ethanol and propylene glycol were mixed. To this solution were dissolved BHT and BHA. Then, the compound of Example 8 was added and the mixture was stirred until dissolution to prepare an external liquid preparation.

### Example 1 Lotion

| | |
|---|---|
| Compound of Example 31 | 0.1 g |
| Polyethylene glycol 400 | 69.9 g |
| Ethanol | 30.0 g |

The above components were weighed and then mixed homogeneously to prepare a lotion.

### Example J Cream

| | |
|---|---|
| Compound of Example 6 | 1% (w/w) |
| Nonoxinol-9 | 5% |
| UNIBASE cream | 94% |

After the above components were weighed, the compound of Example 6 was dissolved in nonoxinol-9. This solution was homogeneously mixed with UNIBASE cream to prepare a cream.

The structural formulae of the compounds of Examples and physical data of Examples are shown at the end of the present specification. Table 4 shows the physical data of the compounds of Examples 4, 5, 7 to 9 and 12 to 19 and Table 5 shows the physical data of the compounds of Examples 21 to 32.

The wave lines mean that no stereochemical prescription is made and the compound of Example 25 is an equilibrated mixture of tautomers.

### EFFECTS OF THE INVENTON

The apoptosis inhibitors of the present invention have inhibited the apoptosis of WC8 cells induced by Fas ligand. In animal models, they have potently inhibited the progress of fulminant hepatitis and have inhibited alopecia due to anticancer agents. The apoptosis inhibitors of the present invention are highly safe and are usable as beneficial drug.

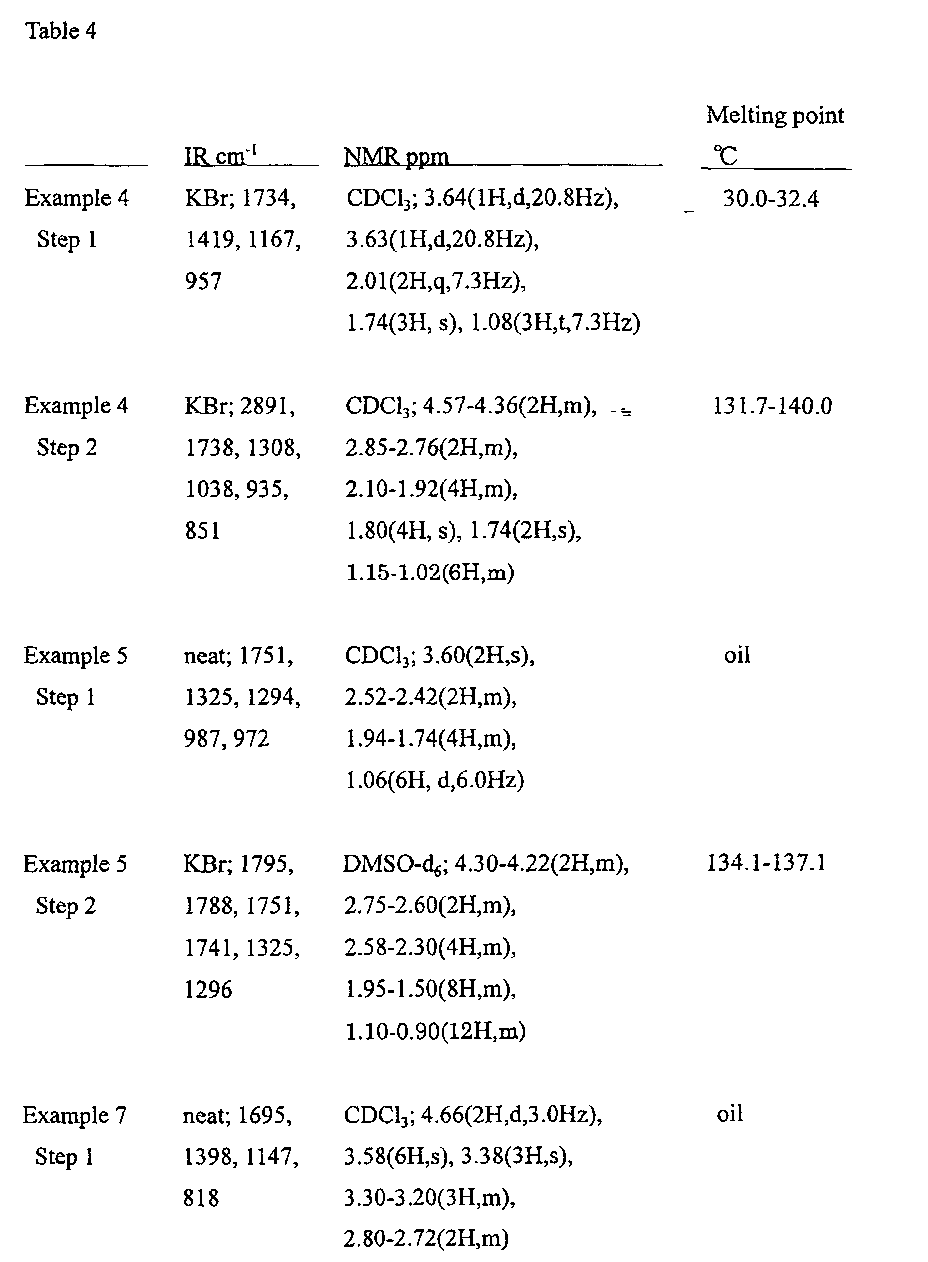

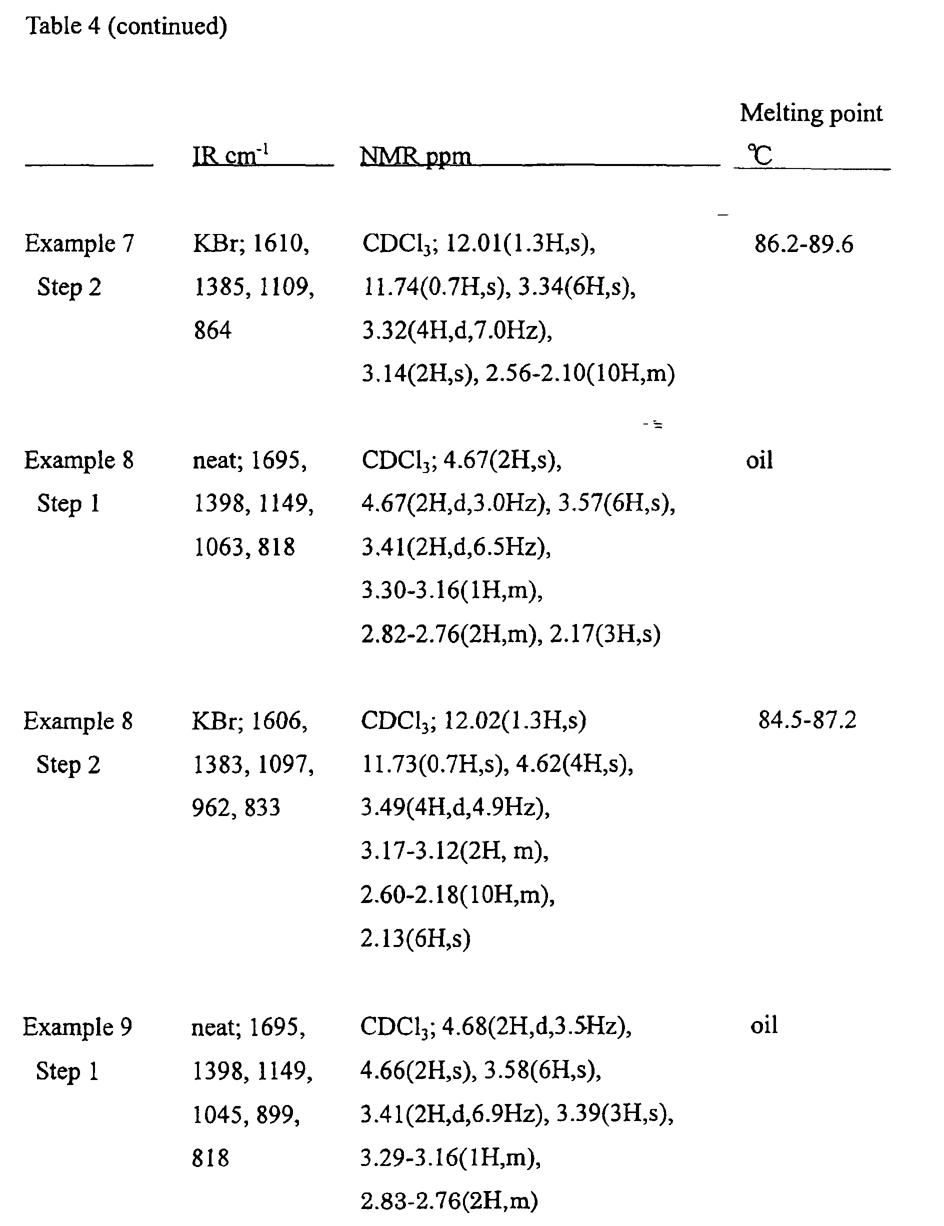

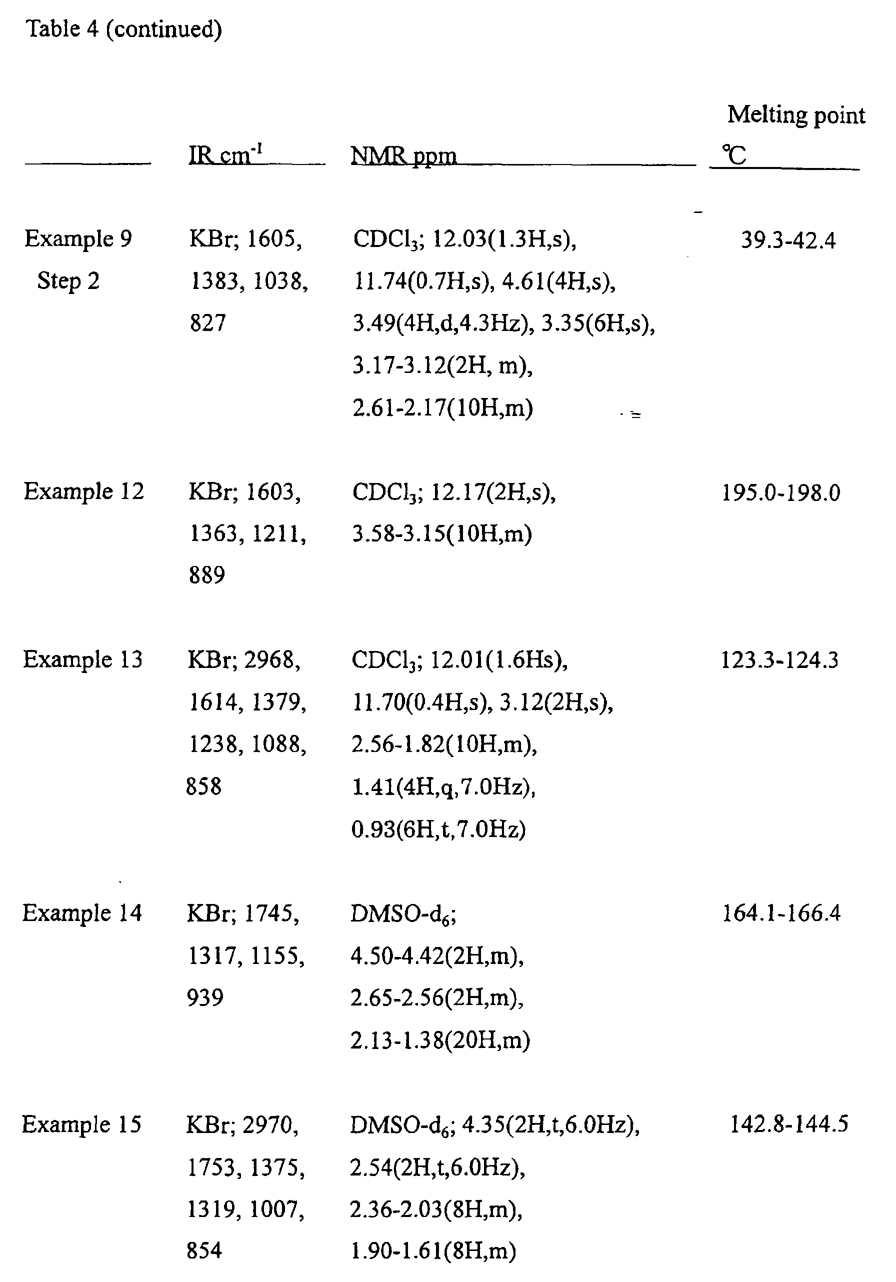

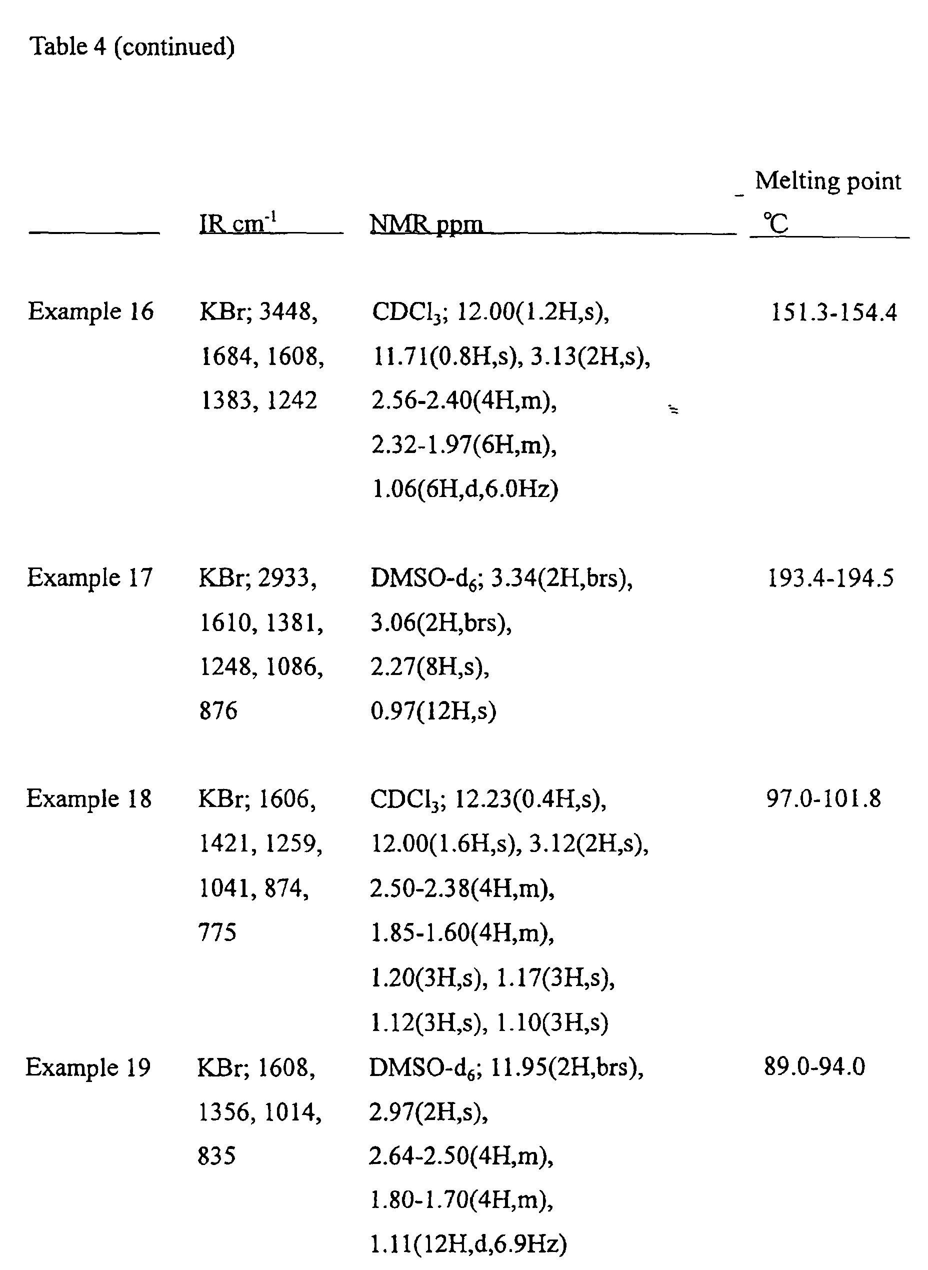

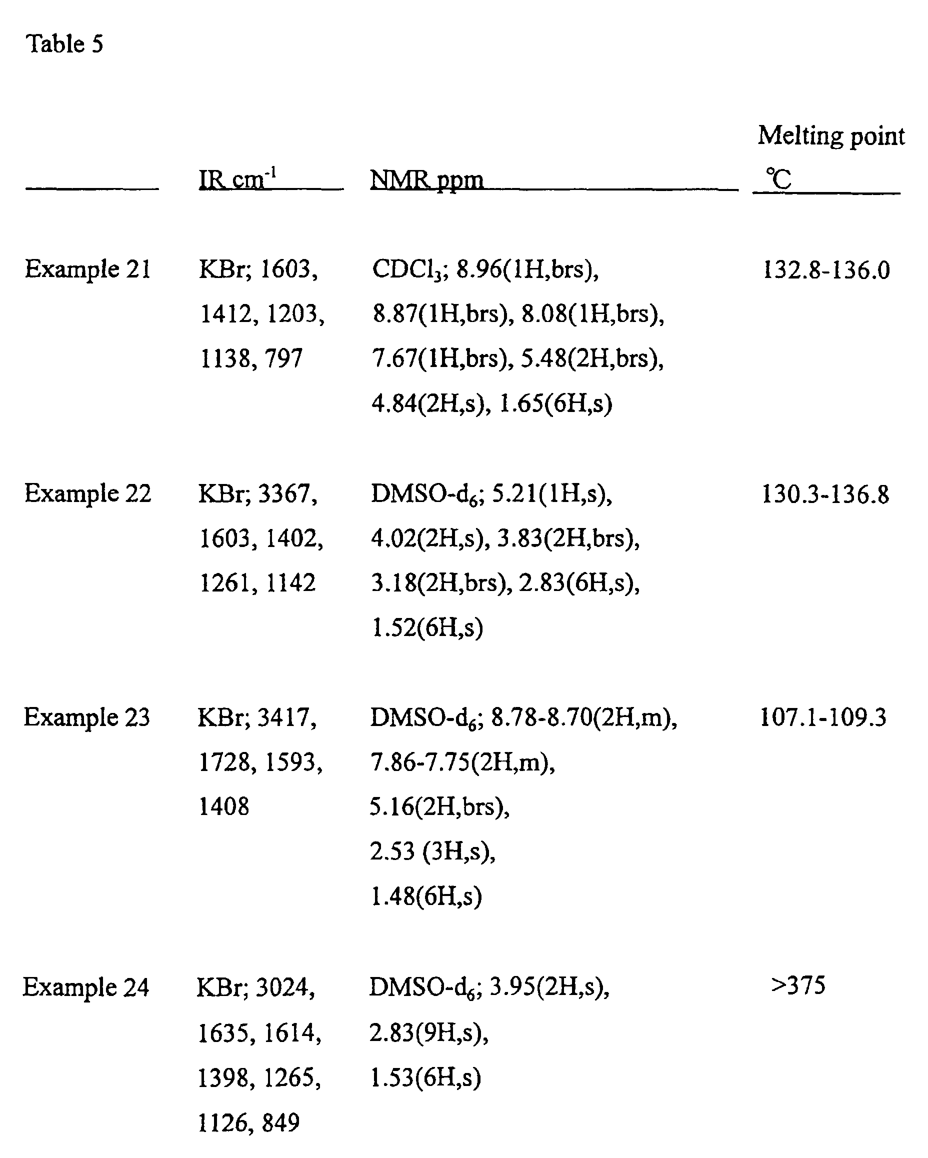

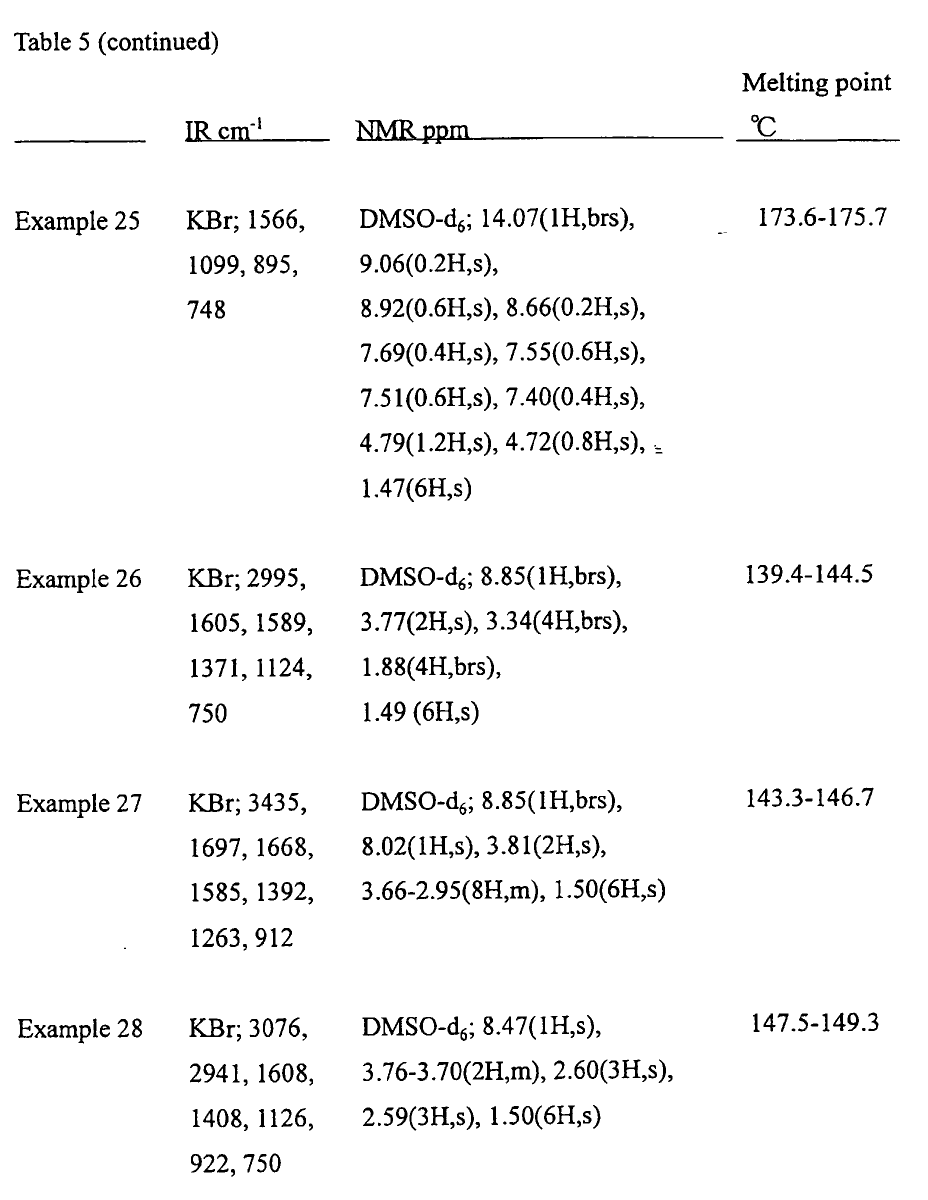

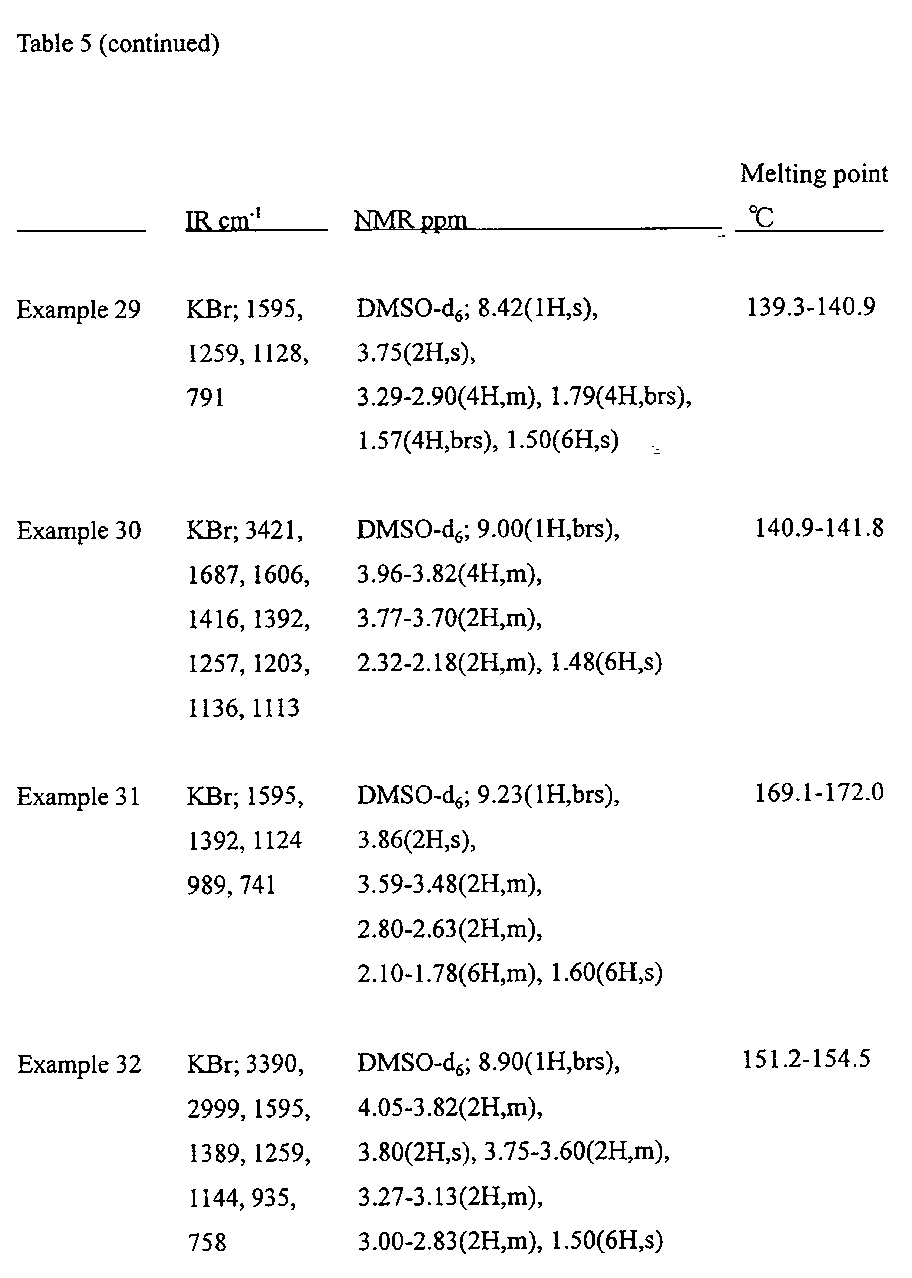

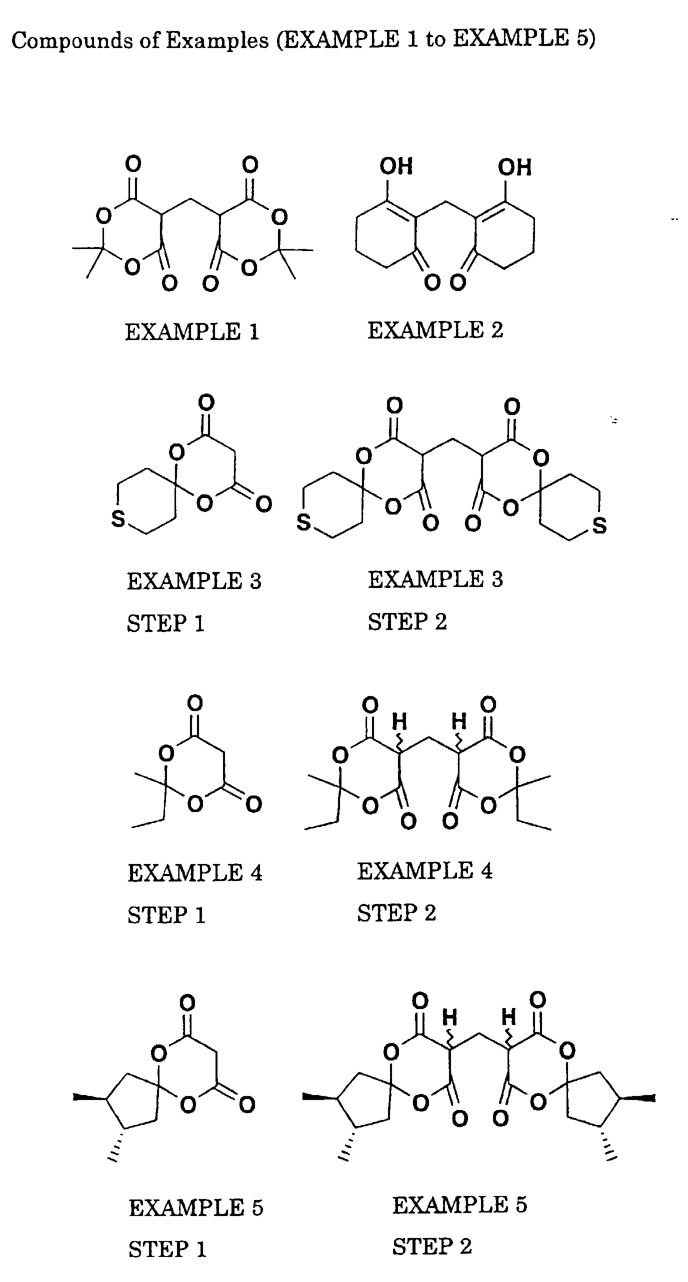

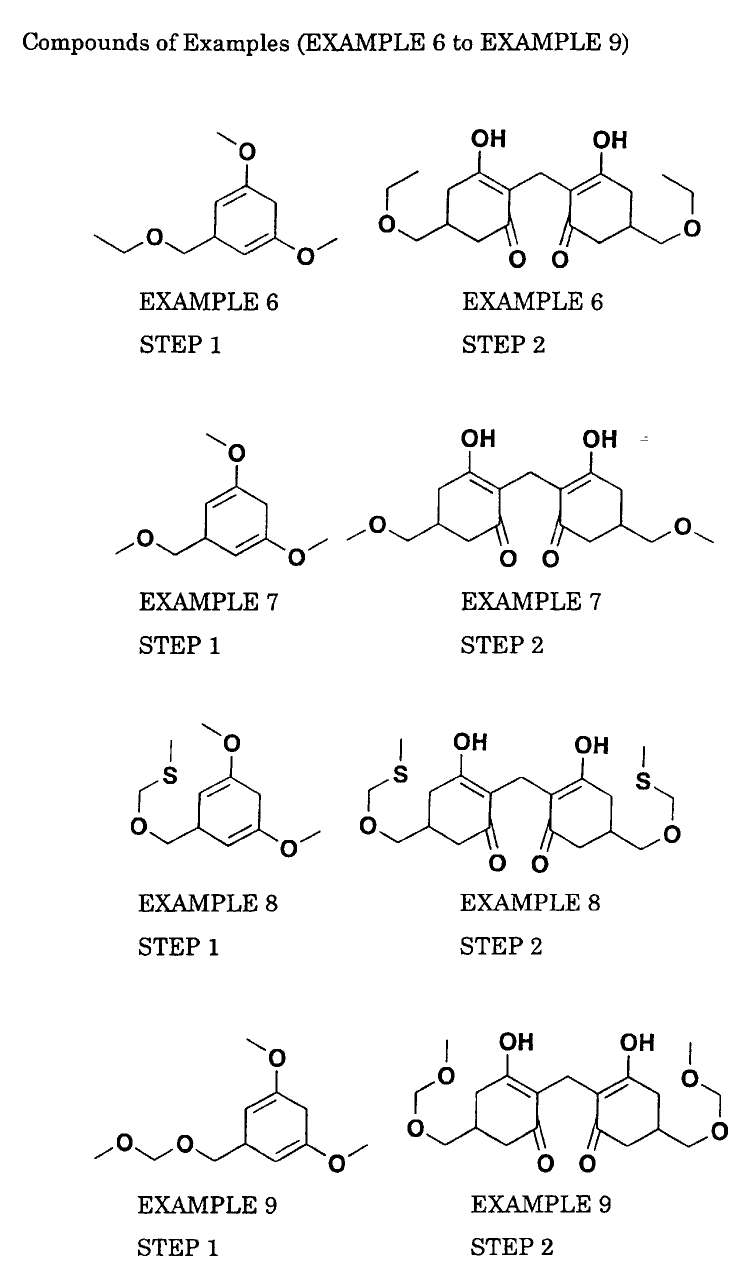

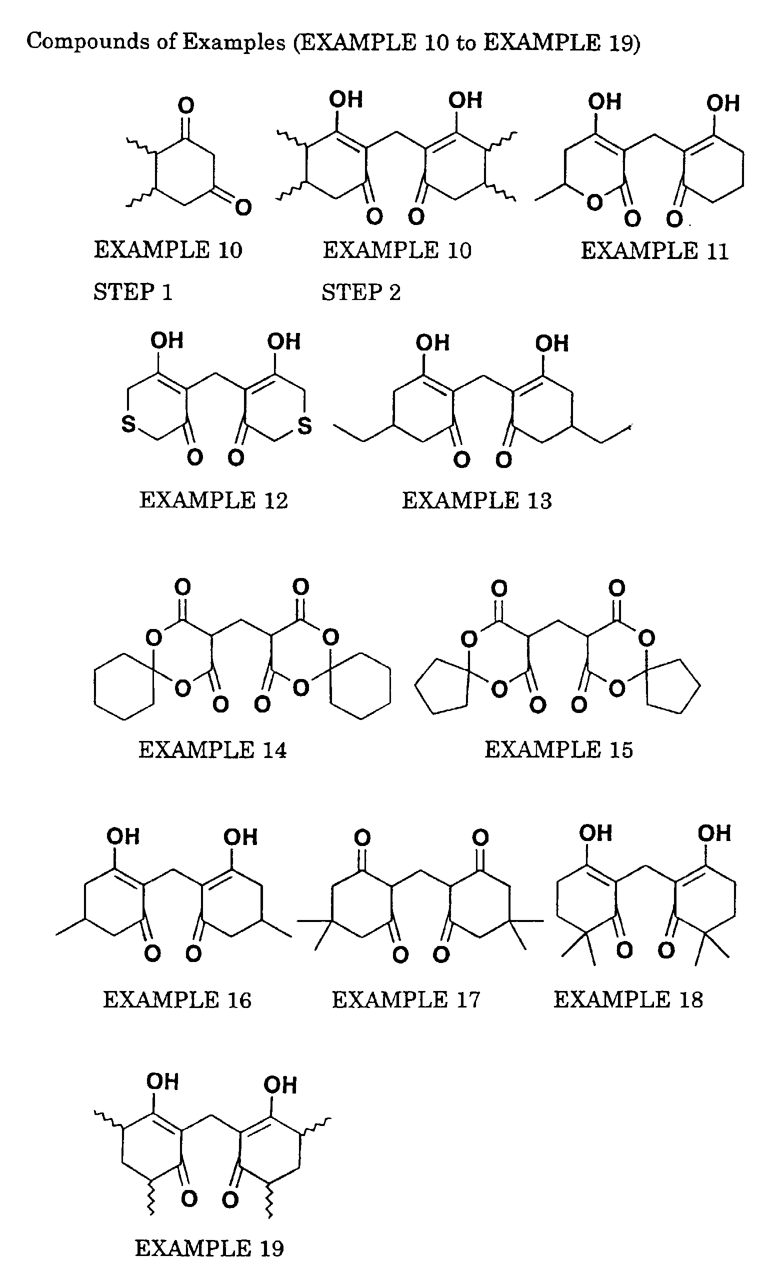

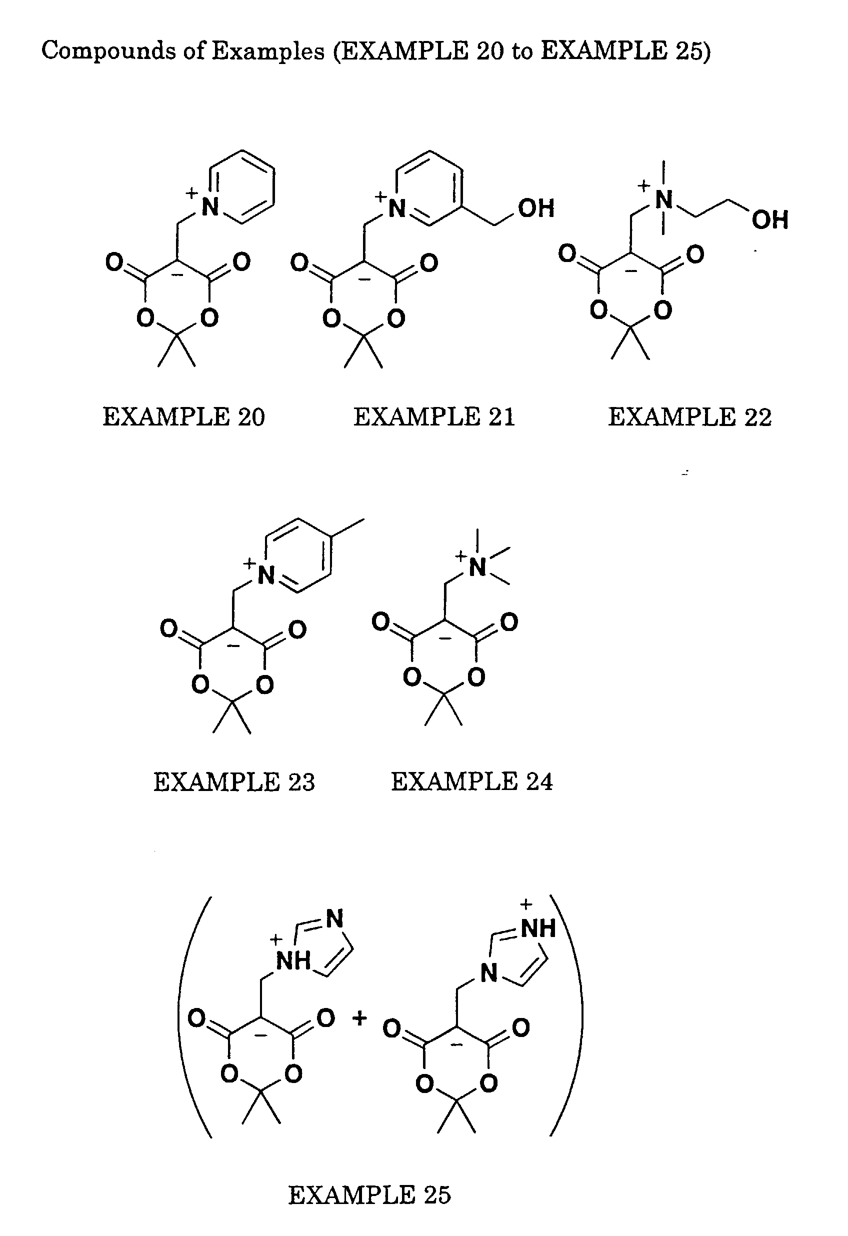

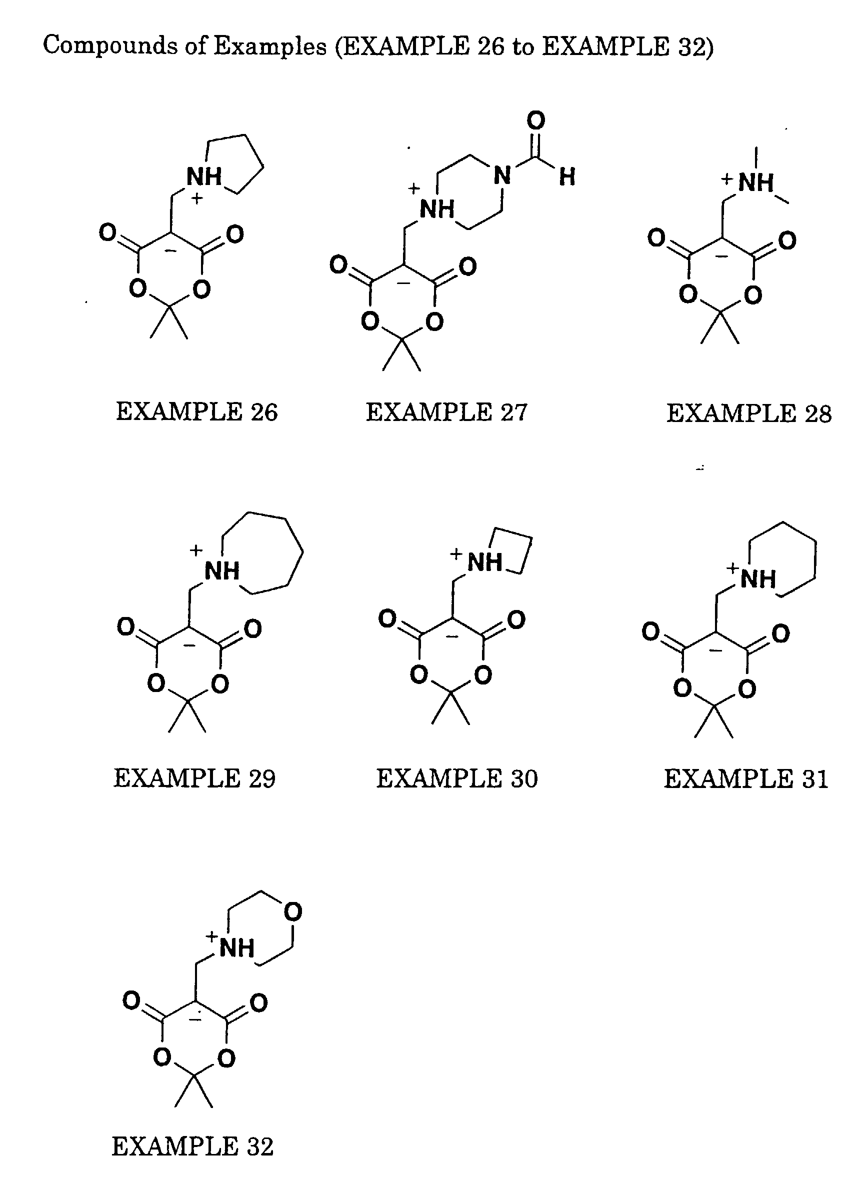

## Claims

1. An apoptosis inhibitor containing a compound of the formula (I): 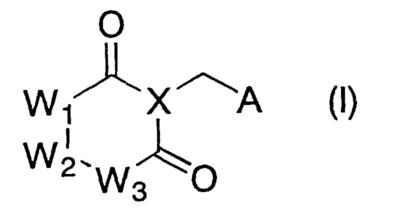 (wherein W₁ represents -O- or -CH(R₁)-, W₂ represents -S- or -CR₂(R₃)-, W₃ represents -O- or CR₄(R₅)-, R₁, R₄, and R₅ each independently represent -H or a straight chain or branched alkyl group having 1 to 6 carbon atoms, R₂ and R₃ each independently represent -H, a straight chain or branched alkyl group having 1 to 6 carbon atoms, a straight chain or branched alkyl group having 1 to 6 carbon atoms substituted by a straight chain or branched alkoxy group having 1 to 6 carbon atoms, or -(CH₂)ₗO(CH₂)ₘY(CH₂)ₙH, or R₂ and R₃ taken together represents an alkylene having 4 to 7 carbon atoms, which may have a branched chain, or -(CH₂)ₚS(CH₂)_{q}-, Y represents O or S, l, m, n, p, and q each independently represent an integer of 1 to 6, X represents CH or C⁻,
A represents 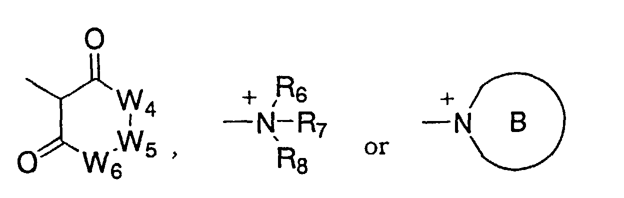 W₄ represents -O- or -CH(R₉)-, W₅ represents -S- or -CR₁₀(R₁₁)-, W₆ represents -O- or -CR₁₂(R₁₃)-, R₆, R₇, and R₈ each independently represent -H, a straight chain or branched alkyl group having 1 to 6 carbon atoms or a straight chain or branched hydroxyalkyl group having 1 to 6 carbon atoms, R₉, R₁₂, and R₁₃ each independently represent -H or a straight chain or branched alkyl group having 1 to 6 carbon atoms, R₁₀ and R₁₁ each independently represent -H, a straight chain or branched alkyl group having 1 to 6 carbon atoms, a straight chain or branched alkyl group having 1 to 6 carbon atoms substituted by a straight chain or branched alkoxy group having 1 to 6 carbon atoms, or -(CH₂)ᵣO(CH₂)ₛZ(CH₂)ₜH, or R₁₀ and R₁₁ taken together represents an alkylene having 4 to 47 carbon atoms, which may have a branched chain, or -(CH₂)ᵤS(CH₂)ᵥ-, Z represents O or S, r, s, t, u, and v each independently represent an integer of 1 to 6, 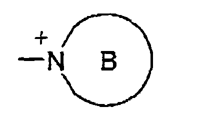 represents saturated or unsaturated, 4- to 7-membered monocyclic heterocyclic onium group having 3 to 6 carbon atoms and 0 or 1 oxygen atom and 1 or 2 nitrogen atom as ring constituting atoms which may have -CH₃, -CH₂OH or -CHO as a substituent on the ring) or salts thereof as an active ingredient.

2. The apoptosis inhibitor as claimed in claim 1, wherein X is CH, A is 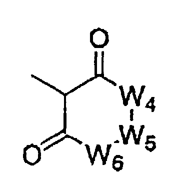 (wherein W₄, W₅, and W₆ have the same meanings as defined in claim 1 above).

3. The apoptosis inhibitor as claimed in claim 1, wherein X is C⁻ , A is 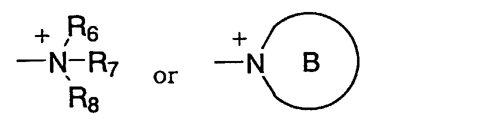 (wherein R₆, R₇, R₈ and 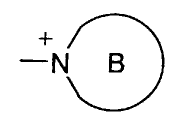 have the same meanings as defined in claim 1 above).

4. A compound of the formula (Ia): 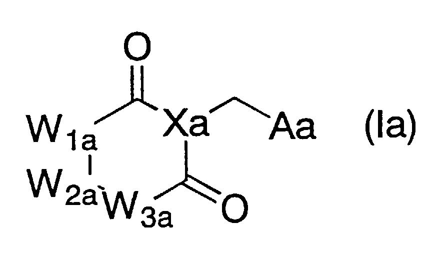 (wherein Xa represents CH or C⁻, Aa represents 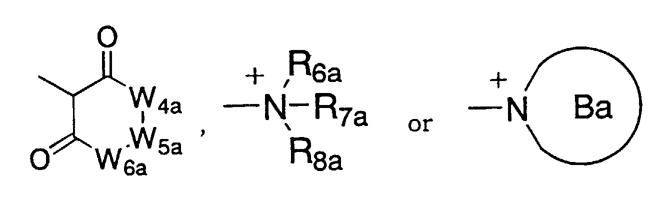 when Xa is CH and Aa is 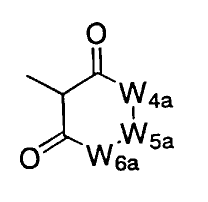 W₁ₐ, W₃ₐ, W₄ₐ, and W₆ₐ represents -O-, W₂ₐ represents -CR₂ₐ(R₃ₐ)-, W₅ₐ represents -CR₁₀ₐ(R₁₁ₐ), R₂ₐ and R₃ₐ, and R₁₀ₐ and R₁₁ₐ, taken together represent -(CH₂)₂S(CH)₂-, or W₁ₐ represents -CH(R₁ₐ)-, W₂ₐ represents -S- or -CH(R₃ₐ)-,W₃ₐ represents -CR₄ₐ(R₅ₐ)-, W₄ₐ represents -CH(R₉ₐ)-, W₅ₐ represents -S- or -CH(R₁₁ₐ)-, W₆ₐ represents CR₁₂ₐ(R₁₃ₐ)-, R₁ₐ, R₄ₐ, R₅ₐ, R₉ₐ, R₁₂ₐ, and R₁₃ₐ each independently represent -CH₂OCH₃, - CH₂OCH₂CH₃, -CH₂OCH₂OCH₃ or -CH₂OCH₂SCH₃, and
when Xa is C⁻, Aa is 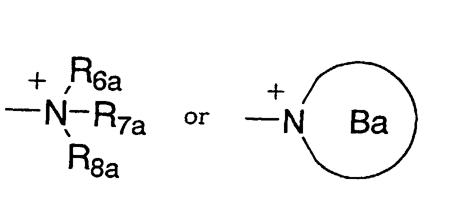 R₆ₐ, R₇ₐ, and R₈ₐ each independently represent a hydrogen atom, an alkyl group having 1 to 6 carbon atoms or a hydroxyalkyl group having 1 to 6 carbon atoms, 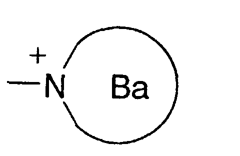 represents a saturated or unsaturated, 4-, 5- or 7-membered monocyclic heterocyclic onium group having 3 to 6 carbon atoms and 0 or 1 oxygen atom and 1 or 2 nitrogen atom as ring constituting atoms) or salts thereof.

5. The compound or salts thereof as claimed in claim 4, wherein Xa is CH and Aa is 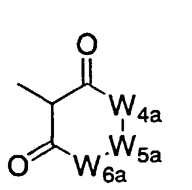 (wherein W₄ₐ, W₅ₐ, and W₆ₐ have the same meanings as defined in claim 4 above).

6. The compound or salts thereof as claimed in claim 4, wherein Xa is C⁻ and Aa is 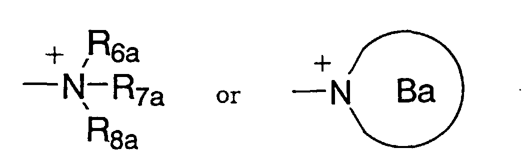 (wherein R₆ₐ, R₇ₐ, R₈ₐ, and 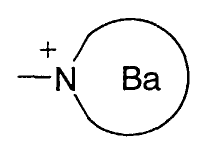 have the same meanings as defined in claim 4 above).

7. A pharmaceutical composition characterized by containing a compound of the formula (I): 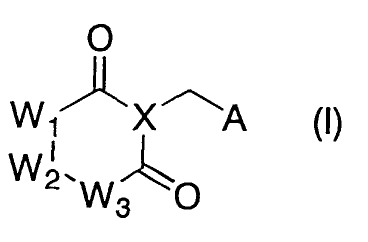 (wherein W₁, W₂, W₃, X, and A have the same meanings as defined in claim 1 above) or salts thereof as an active ingredient.

8. A preventive or therapeutic agent for AIDS, characterized by containing a compound of the formula (I) or salts thereof as an active ingredient.

9. A preventive or therapeutic agent for fulminant hepatitis, characterized by containing a compound of the formula (I) or salts thereof as an active ingredient.

10. A preventive or therapeutic agent for alopecia accompanying use of an anticancer agent, characterized by containing a compound of the formula (I) or salts thereof as an active ingredient.

11. A preventive or therapeutic agent for alopecia accompanying use of an anticancer agent, characterized by containing a Fas-mediated apoptosis inhibitor as an active ingredient.
